# EUROPEAN PATENT SPECIFICATION

(11) **EP 2 398 822 B1**
(45) Date of publication and mention of the grant of the patent: **02.01.2013**
(21) Application number: 10704152.7
(22) Date of filing: 18.02.2010
(51) Int. Cl.: C07K 14/755, A61K 38/37, C12P 21/02

(54) **MODIFICATION OF FACTOR VIII**
MODIFIKATION VON FAKTOR VIII
MODIFICATION DU FACTEUR VIII

(30) Priority: 19.02.2009 EP 09153257; 19.03.2009 US 161510 P
(43) Date of publication of application: 28.12.2011
(73) Proprietor: Novo Nordisk A/S, 2880 Bagsværd (DK)
(72) Inventor: ZUNDEL, Magali, DK-2870 Dyssegaard (DK); PESCHKE, Bernd, DK-2760 Måløv (DK); KARPF, Ditte Maria, DK-3670 Veksø Sjælland (DK); MADSEN, Kjeld, DK-3500 Værløse (DK)
(86) International application number: PCT/EP2010/052022
(87) International publication number: WO 2010/102886

(56) References cited:
- EP-A- 0 379 606
- WO-A-2006/053299
- WO-A-2007/126808
- SAENKO E L ET AL: "Strategies towards a longer acting factor VIII" HAEMOPHILIA 200607 GB, vol. 12, no. SUPPL. 3, July 2006 (2006-07) , pages 42-51, XP002542117 ISSN: 1351-8216 1365-2516
- RAMOS D ET AL: "Chemoenzymatic synthesis of neoglycopeptides: application to an alpha-Gal-terminated neoglycopeptide." THE JOURNAL OF ORGANIC CHEMISTRY 4 MAY 2001, vol. 66, no. 9, 4 May 2001 (2001-05-04), pages 2948-2956, XP002981410 ISSN: 0022-3263
- SATO M ET AL: "Site-specific introduction of sialic acid into insulin" ANGEWANDTE CHEMIE. INTERNATIONAL EDITION, WILEY VCH VERLAG, WEINHEIM, vol. 43, no. 12, 12 March 2004 (2004-03-12), pages 1516-1520, XP002981412 ISSN: 1433-7851

## Description

### Field of the invention

The present invention relates to Factor VIII derivatives, processes for preparing said derivatives and the use of said derivatives in therapy.

### Background of the invention

Factor VIII is an important protein in the blood clotting cascade. A deficiency in Factor VIII causes the blood clotting disease Haemophilia A. Haemophilia A can be treated by administration of Factor VIII to a patient when required. Effective and convenient prophylactic treatment of Haemophilia A with Factor VIII is currently not possible because the plasma half-life of Factor VIII is low. It is therefore difficult to maintain sufficient Factor VIII activity over long periods of time. Therefore, the identification of new Factor VIII derivatives with longer plasma half-life could offer a safe and convenient prophylactic treatment of Haemophilia A.

It is sometimes possible to increase the plasma half-life of a protein by introducing suitable chemical moieties that shield the protein at positions that are important for the clearance of the protein from the blood. It can also be advantageous to introduce chemical moieties into a protein that act as reporter groups. However, if such chemical moieties are introduced non-selectively, then the biological activity of the protein may be reduced or destroyed. Regioselective introduction of chemical moieties into proteins is therefore desirable. However, it is difficult to achieve regioselective introduction of chemical moieties into large proteins, such as Factor VIII.

There is therefore a need for new techniques for regioselectively introducing chemical moieties in to Factor VIII.

### Summary of the invention

The present inventors have surprisingly found that transglutaminase enzymes selectively target a limited number of glutamine residues in Factor VIII. Thus, use of a transglutaminase in the synthesis of Factor VIII derivatives allows regioselective introduction of chemical moieties into Factor VIII. These methods have been used to prepare a novel class of Factor VIII derivatives.

Thus, the present invention relates to a Factor VIII derivative of formula (I): 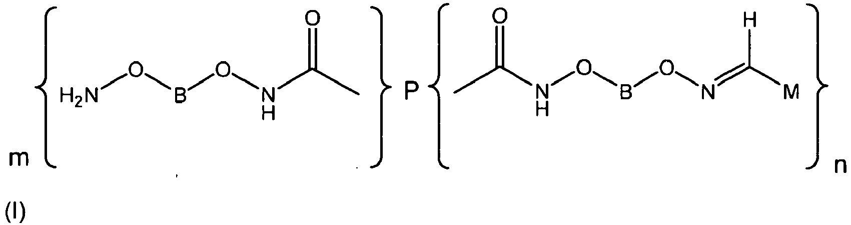 wherein:
B represents C₂ to C₁₀ alkylene;
m represents 0 or an integer from 1 to 19, n represents an integer from 1 to 20, and the sum of m and n is from 1 to 20;
P represents a mono or polyradical of Factor VIII obtained by removing m + n carbamoyl groups from the side chains of glutamine residues in Factor VIII; and
M represents a moiety (M¹) that increases the plasma half-life of the Factor VIII derivative or a reporter moiety (M²);
or a pharmaceutically acceptable salt thereof.

The invention further provides:
- a pharmaceutical composition comprising a Factor VIII derivative as defined above and a pharmaceutically acceptable carrier or diluent;
- a Factor VIII derivative as defined above for use in the treatment of the human or animal body by therapy;
- a Factor VIII derivative as defined above for use in the treatment of Haemophilia A;
- use of a Factor VIII derivative as defined above in the manufacture of a medicament for the treatment of Haemophilia A;
- a method of treating a patient with Haemophilia A, which method comprises the administration to said patient of a therapeutically effective amount Factor VIII derivative as defined above or a pharmaceutical composition as defined above;
- a Factor VIII derivative of formula (II) 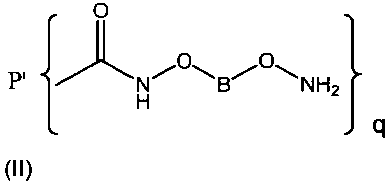 wherein:
   B represents a C₂ to C₁₀ alkylene;
   q represents an integer from 1 to 20; and
   P' represents a mono or polyradical of Factor VIII obtained by removing q carbamoyl groups from the side chains of glutamine residues in Factor VIII,
   or a pharmaceutically acceptable salt thereof;
- a method for preparing a Factor VIII derivative of formula (II) as defined above, which method comprises reacting Factor VIII with a compound of formula (III):

   H₂N-O-B-O-NH₂ (III)

   in the presence of a transglutaminase, wherein B is as defined above; and
- a method for preparing a Factor VIII derivative of formula (I) as defined above, which method comprises reacting a Factor VIII derivative of formula (II) as defined above with an aldehyde of formula (IV): 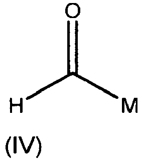
wherein M is as defined above.

### Detailed description of the invention

The present invention provides new Factor VIII derivatives carrying substituents at a limited number of sites on the protein. The regioselective substitution of Factor VIII is controlled by the method of preparation. An important step in the method of the invention is the use of the enzyme transglutaminase (Tgase). Transglutaminase is also known as protein-glutamine-y-glutamyltransferase. Transglutaminase catalyses the general reaction:

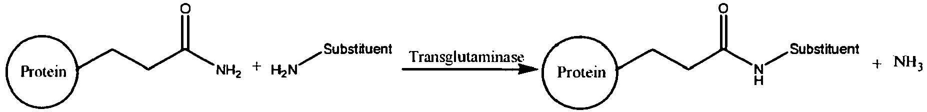

The -CH₂-CH₂-C(O)-NH₂ group on the protein illustrated above is the side chain of a glutamine residue in the protein.

The present inventors have surprisingly found that transglutaminase selectively targets a limited number of glutamine residues in Factor VIII. The sequences of the Factor VIII derivatives contain 64-70 glutamine residues. However, transglutaminase only targets a minority of these glutamine residues. Typically, 1 to 20 glutamine residues are targeted by transglutaminase, preferably 1 to 15, more preferably 1 to 10, and most preferably 1 to 7. Most preferably, the FVIII derivative is B Domain Deleted Factor VIII compound to which a peptide with the sequence of SFSQNSRHPSQNPPVLKRHQR is attached to the C-terminus of the Heavy Chain. This Factor VIII analogue has 66 glutamine residues. The transglutaminase is the transglutaminase from *Streptomyces mobaraense,* and the number of glutamine residues targeted by the enzyme is between 1 and 20.

The first step of the method of present invention involves reacting Factor VIII with a dihydroxylamine compound of formula (III):

H₂N-O-B-O-NH₂ (III)

in the presence of a transglutaminase. The transglutaminase catalyses the reaction of side chains of glutamine residues on Factor VIII with the amine groups on the dihydroxylamine compound of formula (III), to give a Factor VIII derivative of formula (II): 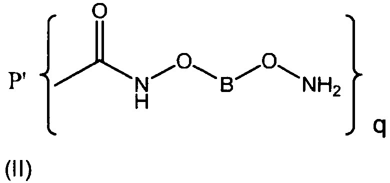 wherein:
B represents C₂ to C₁₀ alkylene;
q represents an integer in the range 1 to 20; and
P' represents a mono or polyradical of Factor VIII obtained by removing q carbamoyl groups from the side chains of glutamine residues in Factor VIII.

In an embodiment B is -CH₂-CH₂-CH₂-.

As will be apparent to one skilled in the art, the formation of the Factor VIII derivative of formula (II) involves the reaction of "q" sides chains of glutamine residues on Factor VIII. Each Factor VIII molecule therefore reacts with "q" molecules of the dihydroxylamine compound.

Typically, this reaction step is carried out in an aqueous solution, preferably a buffered aqueous solution. Suitable buffer solutions are known to those skilled in the art. The temperature of said solution is typically from 0°C to 60°C, preferably from 20°C to 40°C.

The number of modified glutamine residues may be controlled by the concentration of each of the reactants which are, on one hand, FVIII or a FVIII analogue and on the other hand, the bishydroxylamine reagent. Likewise, the concentration of enzyme (measured in activity) and the origin of the transglutaminase can be used to control the extend of reaction, the site or sites of modification and the reaction rate.

The crude product is generally purified by known techniques, such as ion exchange and/or ultrafiltration.

The second step of the method of present invention involves reacting the Factor VIII derivative of formula (II) with an aldehyde of formula (IV):

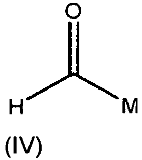

The aldehyde of formula (IV) and the -C(O)-NH-O-B-O-NH₂ moiety or moieties on the Factor VII derivative of formula (II) react to form a Factor VII derivative of formula (I):

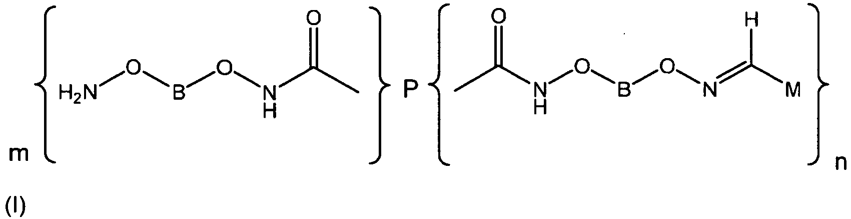

A person skilled in the art can easily determine reactions conditions suitable for the above step. Exact reaction conditions will depend on the nature of the substituent M. Typically said reaction occurs in an aqueous solution, preferably a buffered aqueous solution. Preferably a pH is used suitable for the formation of oximes, where FVIII is stable such as e.g. pH 6.0-8.5, more preferably pH 6.3-7.5. Suitable buffer solutions are known to those skilled in the art. The temperature of said solution is typically from 0°C to 60°C, preferably from 20°C to 40°C. The reaction can be monitored by known techniques, to determine an optimal reaction or incubation time. The crude product is generally purified by known techniques, such as ion exchange and/or ultrafiltration, before subsequent steps.

The Factor VIII derivatives of Formula (II) are useful intermediates in the formation of a Factor VIII derivative of Formula (I).

The sum of m and n in the Factor VIII derivative of formula (I) is equal to q in the Factor VIII derivative of formula (II) it is prepared from. Thus, (i) n of the q -C(O)-NH-O-B-O-NH₂ moieties in the Factor VIII derivative of formula (II) react with the aldehyde of formula (IV) and (ii) m of the q -C(O)-NH-O-B-O-NH₂ moieties in the Factor VIII derivative of formula (II) do not react with the aldehyde of formula (IV). Each molecule of the Factor VIII derivative of formula (II) therefore reacts with n molecules of the aldehyde of formula (IV).

In the compounds of formula (II), q represents an integer from 1 to 20. Typically q represents an integer from 1 to 15. Preferably q represents an integer from 1 to 10. More preferably q represents an integer from 1 to 6.

In the compounds of formula (I), m represents 0 or an integer from 1 to 19, n represents an integer from 1 to 20, and the sum of m and n is from 1 to 20. Preferably m represents 0 or an integer from 1 to 14, n represents an integer from 1 to 15, and the sum of m and n is from 1 to 15. More preferably m represents 0 or an integer from 1 to 9, n represents an integer from 1 to 10, and the sum of m and n is from 1 to 10. Most preferably m represents 0 or an integer from 1 to 5, n represents an integer from 1 to 6, and the sum of m and n is from 1 to 6.

As used herein, a pharmaceutically acceptable salt is a salt with a
pharmaceutically acceptable acid or base. Pharmaceutically acceptable acids include both inorganic acids such as hydrochloric, sulphuric, phosphoric, diphosphoric, hydrobromic or nitric acid and organic acids such as citric, fumaric, maleic, malic, ascorbic, succinic, tartaric, benzoic, acetic, methanesulphonic, ethanesulphonic, benzenesulphonic or p-toluenesulphonic acid. Pharmaceutically acceptable bases include alkali metal (e.g. sodium or potassium) and alkali earth metal (e.g. calcium or magnesium) hydroxides and organic bases such as alkyl amines, aralkyl amines and heterocyclic amines.

The Factor VIII activity of the derivatives discussed above is typically substantially the same as the activity of activated human Factor VIII. "FVIII activity" is defined as the ability to function in the coagulation cascade, induce the formation of Factor Xa via interaction with Factor IXa on an activated platelet, and support the formation of a blood clot. Factor VIII activity can be assessed *in vitro* by techniques such as clot analysis, as described in e.g. Manucci and Tripodi, "Factor VIII clotting activity". E. C. A. T. assay procedures, London: Kluwer Academic Publishers, 1999; endogenous thrombin potential analysis, as described in Hemker et al., "The thrombogram: monitoring thrombin generation in platelet- rich plasma.", Thrombosis and haemostasis, vol. 83:589-591; and other techniques known to people skilled in the art.

Thus, the Factor VIII derivatives of the invention will typically be screened to assess whether they have maintained substantially the same activity as activated human Factor VIII.

As used herein, Factor VIII activity substantially the same as the activity of activated human Factor VIII means that the Factor VIII activity is at least 20%, at least 30%, at least 40%, at least 50%, at least 60 %, at least 70 %, at least 80%, at least 90% such as at least 100% of that of human Factor VIII. The Factor VIII activity is in particular about 50 to about 75%, about 75 to about 85%, about 85 to about 95% and even more than 100% of that of human Factor VIII.

### Transglutaminase

The term "transglutaminase" as used herein refers to enzymes in class EC 2.3.2.13. Examples of a useful transglutaminase include a microbial transglutaminase, typically from *Streptomyces mobaraense, Streptomyces cinnamoneum* and *Streptomyces griseo- carneum* (all disclosed in US Patent No. 5,156,956, which is incorporated herein by reference), *Streptomyces lavendulae* (disclosed in US Patent No. 5,252,469, which is incorporated herein by reference) or *Streptomyces ladakanum* (JP-A-2003199569, which is incorporated herein by reference). It should be noted that members of the former genus *Streptoverticillium* are now included in the genus *Streptomyces* [Kaempfer, J. Gen. Microbiol., 137, 1831-1892, 1991].

Other examples of a useful microbial transglutaminase have been isolated from Ba*cillus subtilis* (disclosed in US 5,731,183, which is incorporated herein by reference) and from various *Myxomycetes.* Other examples of a useful microbial transglutaminase are those disclosed in WO 96/06931 (e.g. transglutaminase from *Bacilus lydicus*) and WO 96/22366, both of which are incorporated herein by reference.

A useful non-microbial transglutaminase includes a guinea-pig liver transglutaminase, and a transglutaminase from various marine sources like the flat fish *Pagrus major* (disclosed in EP-A-0555649, which is incorporated herein by reference), and the Japanese oyster *Crassostrea gigas* (disclosed in US Patent No. 5,736,356, which is incorporated herein by reference).

Other transglutaminases which should be mentioned are human transglutaminases TG2, TG3, TG7 or FXIII.

Other useful non-microbial transglutaminases are the human transglutaminases TG1 and TG6.

A transglutaminase from *Streptomyces mobaraense* is preferred.

### Factor VIII

The mature Factor VIII molecule consists of 2332 amino acids which can be grouped into three homologous A domains, two homologous C domains and a B Domain which are arranged in the order: A1-A2-B-A3-C1-C2. During its secretion into plasma Factor VIII is processed intracellularly into a series of metal-ion linked heterodimers as single chain Factor VIII is cleaved at the B-A3 boundary and at different sites within the B-domain. This processing leads to a heavy chain consisting of the A1, the A2 and various parts of the B-domain which has a molecular size ranging from 90 kDa to 200 kDa. The heavy chains are bound via a metal ion to the light chain, which consists of the A3, the C1 and the C2 domain. In plasma, this heterodimeric Factor VIII binds with high affinity to von Willebrand Factor (VWF), which protects it from premature catabolism. The half-life of non-activated Factor VIII bound to vWF is about 12 hours in plasma.

During the blood coagulation process, Factor VIII is activated via proteolytic cleavage by Factor Xa and thrombin at amino acids Arg372 and Arg740 within the heavy chain and at Arg1689 in the light chain resulting in the release of von Willebrand Factor and generating the activated Factor VIII heterotrimer which will form the tenase complex on phospholipid surfaces with Factor IXa and Factor X provided that Ca²⁺ is present. The heterotrimer consists of the A1 domain, a 50 kDa fragment, the A2 domain a 43 kDa fragment and the light chain (A3-C1-C2), a 73 kDa fragment. Thus the active form of Factor VIII (Factor VIIIa) consists of an A1-subunit associated through the divalent metal ion linkage to a thrombin-cleaved A3-C1-C2 light chain and a free A2 subunit relatively loosely associated with the A1 and the A3 domain.

A Factor VIII molecule consisting of the heavy chain (HC) and light chain (LC) of Factor VIII connected with a small linker derived from the B-domain (B-domain deleted Factor VIII or BDD-FVIII) retains the biological activity of full length (native) Factor VIII.

As used herein, the term "Factor VIII" includes any Factor VIII polypeptide that is therapeutically useful, e.g. effective in preventing or treating bleeding. This includes, without limitation, wild-type human Factor VIII, hybrid human/porcine Factor VIII, B-domain deleted human Factor VIII and partially B-domain deleted human Factor VIII.

The term "Factor VIII" is intended to encompass, without limitation, polypeptides having the amino acid sequence as described in Toole et al., Nature 1984, 312: 342-347 (wild-type human Factor VIII), as well as wild-type Factor VIII derived from other species, such as, e.g., bovine, porcine, canine, murine, and salmon Factor VIII. It further encompasses natural allelic variations of Factor VIII that may exist and occur from one individual to another. Also, degree and location of glycosylation or other post-translation modifications may vary depending on the chosen host cells and the nature of the host cellular environment. The term "Factor VIII" is also intended to encompass uncleaved (zymogen) forms, as well as those that have been proteolytically processed to yield their respective bioactive forms, which may be designated Factor VIIIa.

The term "Factor VIII" is intended to encompass polypeptides with a slightly modified amino acid sequence, for instance, polypeptides having a modified N-terminal end including N-terminal amino acid deletions or additions, and/or polypeptides that have been chemically modified relative to human Factor VIII. The term "Factor VIII" is intended to include variants of Factor VIII, whether exhibiting substantially the same or better bioactivity than wild-type Factor VIII, or, alternatively, exhibiting substantially modified or reduced bioactivity relative to wild-type Factor VIII, include, without limitation, polypeptides having an amino acid sequence that differs from the sequence of wild-type Factor VIII by insertion, deletion, or substitution of one or more amino acids.

Non-limiting examples of Factor VIII include plasma-derived human Factor VIII as described, e.g., in Fulcher et al.; Proc. Acad. Nat. Sci. USA 1982; 79:1648-1652 , and Rotblat et al.; Biochemistry 1985; 24:4294-4300 , and plasma-derived porcine FVIII as described, e.g., in Fass et al.; Blood 1982; 59: 594-600 and Knutson et al.; Blood 1982; 59: 615-624. Non-limiting examples of Factor VIII sequence variants are described, e.g., in Lollar et al.; Blood 2000; 95(2): 564-568 (hybrid porcine/human FVIII polypeptides) and Lollar et al.; Blood 2001; 97(1): 169-174.

The cloning of the cDNA for Factor VIII (Wood, W.I., et al. (1984) Nature 312, 330-336 ; Vehar, G.A., et al. (1984) Nature 312, 337-342) made it possible to express Factor VIII recombinantly leading to the development of several recombinant Factor VIII products, which were approved by the regulatory authorities between 1992 and 2003. The fact that the central B domain of the Factor VIII polypeptide chain residing between amino acids Arg-740 and Glu-1649 does not seem to be necessary for full biological activity has also led to the development of a B-domain deleted Factor VIII. See also Kjalke M, Heding A, Talbo G, Persson E, Thomsen J and Ezban M (1995), "Amino acid residues 721-729 are required for full Factor VIII activity". Eur. J. Biochem: 234: 773-779 . Factor VIII as used herein includes all variants of Factor VIII, including those in which one or more domains or regions have been deleted.

The positions of the glutamine residues reacting under catalysis of transglutaminase may be determined by direct digest with suitable enzymes such as e.g. trypsin, followed by peptide mapping or by peptide mapping with a reporter group such as e.g. biotin or a fluorescence group such as e.g. Alexa 488. Typically, when Factor VIII is reacted with transglutaminase from *Streptomyces mobaraense,* most of the glutamine residues shown in formula (I) are from the side chains of glutamine residues in the heavy chain of Factor VIII. Preferably said glutamine residues are mostly in the A1 domain.

As used herein, the term carbamoyl group refers to the radical -C(O)-NH₂, as is present in, for example, the side chain of a glutamine residue. As used herein, the term "mono or polyradical of Factor VIII obtained by removing n + m or q carbamoyl groups from the side chains of glutamine residues in Factor VIII" means that n + m or q -C(O)-NH₂ groups are formally removed from side chains of glutamine residues. As the skilled person will understand, the use of these terms does not indicate that a carbon-carbon bond is broken in the glutamine residues, merely that the definition of "mono or polyradical of Factor VIII" used herein does not include the carbamoyl portions of one or more glutamine residues. This is illustrated below:

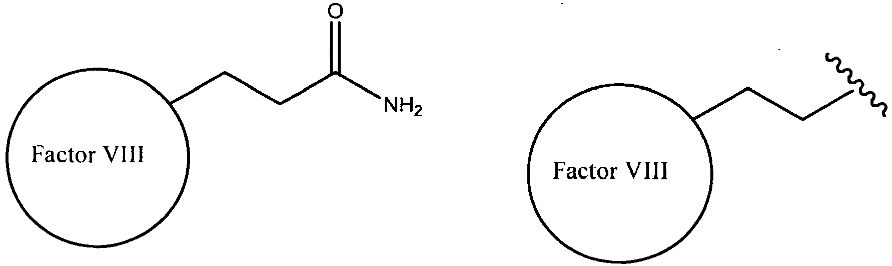

The diagram on the left shows Factor VIII molecule with a glutamine side chain. The diagram on the right shows a "mono of Factor VIII obtained by removing one carbamoyl group from the side chain of a glutamine residue in Factor VIII".

### Moieties (M¹) that increase the plasma half-life of the Factor VIII derivative

The reaction of the Factor VIII derivative of formula (II) with an aldehyde of formula (IV) introduces a moiety M into Factor VIII. In an embodiment, M is a moiety (M¹) that increases the plasma half-life of the Factor VIII derivative.

Factor VIII has a number of clearance sites. As used herein, the term "clearance site" is defined as a region on the Factor VIII molecule that is recognized by the physiological machinery responsible for degradation of the protein. Thus, the half-life of Factor VIII can be increased by disrupting said clearance sites by introducing a substituent M₁. A "disrupted clearance site" is defined as a clearance site on the Factor VIII molecule that exhibits reduced binding to its cognate receptor or interaction partner as a result of above-mentioned modification.

Thus, the plasma half-life of Factor VIII can be improved by introducing one or more moieties into Factor VIII that disrupt clearance sites. Such moieties typically hide, mask or eclipse one or more clearance sites on Factor VIII. Thus, in one embodiment, the invention provides a Factor VIII derivative with an improved plasma half-life. The improvement is with respect to the corresponding unmodified Factor VIII.

The plasma half-life of Factor VIII or a Factor VIII derivative is determined by measuring the *in vivo* plasma half-life. Human factor VIII has a plasma half-life of about 12-14 hours. *"In vivo* plasma half life" is the time at which 50% of the Factor VIII or a Factor VIII derivative circulates in the plasma or bloodstream prior to being cleared. Determination of plasma half-life is typically simpler than determining functional half-life and the magnitude of plasma half-life is usually a good indication of the magnitude of functional in vivo half-life. Alternative terms to plasma half-life include serum half-life, circulating half-life, circulatory half-life, serum clearance, plasma clearance, and clearance half-life.

The term "increased" as used in connection with the plasma half-life is used to indicate that the relevant half-life of the Factor VIII derivative is statistically significantly increased relative to that of the unmodified Factor VIII, as determined under comparable conditions. For instance the relevant half-life may be increased by at least about 25%, such as by at lest about 50%, e.g., by at least about 100%, 150%, 200%, 250%, or 500%. In one embodiment, the Factor VIII derivatives of the present invention exhibit an increase in half-life of at least about 5 hours, preferably at least about 24 hours, more preferably at least about 72 hours, and most preferably at least about 7 days, relative to the half-life of the parent Factor VIII.

The term "parent Factor VIII" as used herein refers to the specific Factor VIII from which the Factor VIII derivative in question is derived.

Measurement of *in vivo* plasma half-life can be carried out in a number of ways as described in the literature. An increase in *in vivo* plasma half-life may be quantified as a decrease in clearance (CL) or as an increase in mean residence time (MRT). Factor VIII derivatives of the present invention for which the CL is decreased to less than 70%, such as less than 50%, such than less than 20%, such than less than 10% of the CL of the parent Factor VIII as determined in a suitable assay is said to have an increased *in vivo* plasma half-life. Factor VIII derivatives of the present invention for which MRT is increased to more than 130%, such as more than 150%, such as more than 200%, such as more than 500% of the MRT of the parent Factor VIII in a suitable assay is said to have an increased *in vivo* plasma half-life. Clearance and mean residence time can be assessed in standard pharmacokinetic studies using suitable test animals. It is within the capabilities of a person skilled in the art to choose a suitable test animal for a given protein. Tests in human, of course, represent the ultimate test. Typically, and as an example, the mice, rats, dogs, monkeys or pigs are in injected with the compound of interest. The amount injected depends on the test animal. Subsequently, blood samples are taken over a period of one to five days as appropriate for the assessment of CL and MRT. The blood samples are conveniently analysed by ELISA techniques.

M¹ typically comprises one or more hydrophilic polymer or plasma protein binders. The polymer can either be a chemical polymer usch as e.g. a polyethyleneglycol (PEG) moiety or the polymer can be a biopolymer such as e.g. a polysaccharide, polysialic acid moieties, hyaluronic acid moieties, or polypeptides. An example for a polysaccharide is polysialic acid. An example for a polypeptide consisting of one type of amino acid is poly-Gly. Polypeptides consisting of different amino acids may also be used as examples of the invention. Preferably M¹ comprises either (a) one PEG moiety, polysialic acid polypeptide or plasma protein binder, (b) one PEG moiety and one plasma protein binder, (c) one PEG moiety and one polypeptide, (d) one polypeptide and one plasma protein binder, (e) one polysialic acid moiety and one plasma protein binder, or (f) one polysialic acid moiety and one polypeptide.

The term "PEG" as used herein refers to poly(ethylene glycol), also known as poly(ethylene oxide) (PEO) or polyoxyethylene (POE), are polyethers. PEG is prepared by polymerization of ethylene oxide and are commercially available over a wide range of molecular weights from 300 g/mol to 10,000,000 g/mol.

Different forms of PEG are also available dependent on the initiator used for the polymerization process. The most common form of PEG is a monofunctional methyl ether PEG (methoxypoly(ethylene glycol)), abbreviated mPEG.

PEGs are also available with different geometries, such as linear, and branched PEGs.

PEG has the structure HO-(CH₂-CH₂-O-)ₙ-H, the molecular formula C₂ₙH₄ₙ₊₂Oₙ₊₁, and the CAS number [25322-68-3]. The molar mass of course depends on n.

The numbers that are often included in the names of PEGs indicate their average molecular weights, e.g. a PEG with n=80 would have an average molecular weight of approximately 3500 daltons and would be labeled PEG 3500.

Most PEGs include molecules with a distribution of molecular weights, i.e. they are polydisperse. The size distribution can be characterized statistically by its weight average molecular weight (Mw) and its number average molecular weight (Mn), the ratio of which is called the polydispersity index (Mw/Mn) (see e.g. "Polymer Synthesis and Characterization", J. A. Nairn, University of Utah, 2003). Mw and Mn can be measured by mass spectroscopy.

The polydispersity index is accordingly a number which is greater than or equal to one, and it may also be estimated from Gel Permeation Chromatographic data. When the polydispersity index is 1, the product is monodisperse and is thus made up of compounds with a single molecular weight. When the polydispersity index is greater than 1 the polymer is polydisperse, and the polydispersity index tells how broad the distribution of polymers with different molecular weights is. The polydispersity index typically increases with the molecular weight of the PEG or mPEG.

For the present purposes, the terms "PEG" and "Peg" are used interchangeably and basically mean a radical or diradical comprising the structure 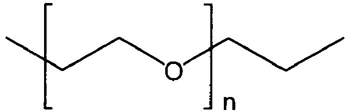 wherein n is an integer larger than 1.

The term PEG is intended to indicate poly(ethylene glycol) as well as poly(ethylene glycol) monoalkyl ether, wherein alkyl indicates C₁₋₆ alkyl, such as methyl, ethyl, propyl, butyl, pentyl and hexyl. Accordingly, in a preferred embodiment, Peg for use according to the invention is represented by the following formula: 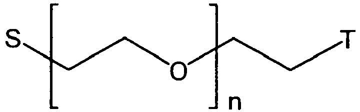 in which n is an integer larger than 1, and S and T independently designates alkyloxy, hydroxy, or is absent. As explained above, a compound of this formula in which S designates methyloxy and T is absent is also referred to as mPEG.

The molecular weight of the PEG for use according to the invention preferably is between approximately 100 Da and approximately 1000000 Da. The molecular weight of the Peg in kDa may be indicated in parentheses. By way of example, mPEG(30k) indicates poly(ethylene glycol) monomethyl ether with a molecular weight of approximately 30 kDa. This polymer may, by the way, be composed of approximately 680 ± 100 ethylene glycol units. As another example, in mPEG(4k) n is 90 and the molecular weight is 3991 Da, i.e. approx 4 kDa. Likewise, mPEG(20k) has an average molecular weight of 20 kDa and an average n of 454.

The PEG for use according to the present invention is linear or branched. In particular embodiments the PEG for use according to the invention is a) polydisperse, or b) monodisperse. In particular embodiments, the polydispersity index of the Peg for use according to the invention is i) below 1.06, ii) below 1.05, iii) below 1.04, iv) below 1.03, or v) between 1.02 and 1.03.

The polysialic acid present in M¹ are preferably homopolymer of N-acetylneuraminic acid with α(2→8) ketosidic linkages (colominic acid), with molecular weight from 8 to 100kD, preferably 20 to 40kD.

The polypeptides present in M¹ are preferably plasma proteins. The term "plasma protein" as used herein refers to albumins, antibodies and fibrinogens, preferably albumins and antibodies.

The term "albumin" as used herein refers to serum albumin from blood serum, and includes human serum albumin as well as serum albumin from other sources. The term "albumin" as used herein includes any derivatives of albumin or modified versions of albumin.

The antibody can be a human antibody or a chimeric antibody. It is preferably a monoclonal antibody. Preferably the antibody is an IgG1 (*e.g*. IgG1,□), IgG3 (*e.g*. IgG3, □and IgG4 (*e.g*. IgG4,□) antibody. However, other antibody isotypes are also encompassed by the invention, including IgG2, IgM, IgA1, IgA2, secretory IgA, IgD, and IgE. Suitable antigen-binding fragments of such antibodies include Fab, F(ab')2, Fv, single chain Fv fragments or bispecific antibodies. Furthermore, the antigen-binding fragments include binding-domain immunoglobulin fusion proteins comprising (i) a binding domain polypeptide (such as a heavy chain variable region or a light chain variable region) that is fused to an immunoglobulin hinge region polypeptide, (ii) an immunoglobulin heavy chain CH2 constant region fused to the hinge region, and (iii) an immunoglobulin heavy chain CH3 constant region fused to the CH2 constant region. Such binding-domain immunoglobulin fusion proteins are further disclosed in US 2003/0118592 and US 2003/0133939. Alternatively, a fragment can comprise the constant region of an antibody; thus a fragment can be a Fc fragment or part thereof.

The term "plasma protein binder" as used herein refers to any moiety capable of binding to a plasma protein, particularly albumin. A moiety that binds to albumin is an "albumin binder". The ability of a compound to bind to albumin may be determined as described in J. Med. Chem, 43, 2000, 1986-1992, which is incorporated herein by reference. In the present context, a compound is defined as binding to albumin if Ru/Da is above 0.05, such as above 0.10, such as above 0.12 or even above 0.15. Albumin binders are typically highly hydrophobic molecules, preferably derived from fatty acids. Thus, an albumin binder will preferably comprises a -(CH₂)₁₂- moiety.

A preferred moiety (M₁) comprising a PEG moiety is: 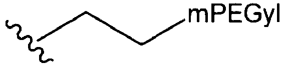 where mPEGyl is polydisperse and has a molecular weight of approximately 20kDa

A preferred moiety (M₁) comprising an albumin binder is:

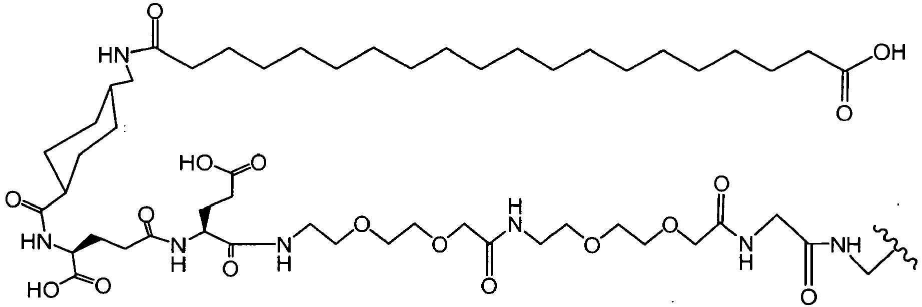

A preferred moiety (M₁) comprising a PEG moiety and an albumin binder is:

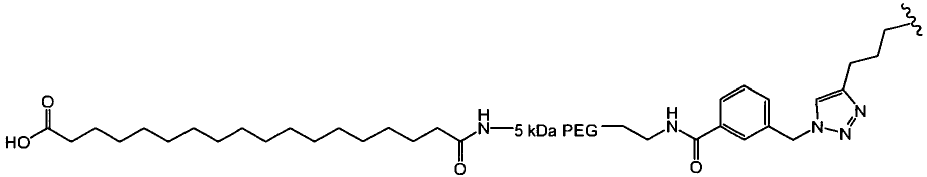

### Reporter moieties (M²)

The reaction of the Factor VIII derivative of formula (II) with an aldehyde of formula (IV) introduces a moiety M into Factor VIII. In an embodiment, M is a reporter moiety (M²). A Factor VIII derivative of formula (II) carrying one or more reporter moieties (M²) typically has substantially the same activity as activated human factor VIII. The term substantially the same activity as activated human factor VIII" has the meaning defined above.

The reporter moieties (M²) may comprise any suitable label which allows the Factor VIII derivative to be detected. Suitable labels are well known to those skilled in the art and include biotin; fluorescent markers such as fluorescein radicals, rhodamine radicals, Texas Red ® radicals, Alexa Fluor ® dyes such as Alex Fluor 488 and phycobili protein radicals; radioisotopes, e.g. Cu-64, Ga67, Ga-68, Zr-89, Ru-97, Tc-99, Rh-105, Pd-109, In-111, I-123, I-125, I-131, Re-186, Re-188, Au-198, Pb-203, At-211, Pb-212 and Bi-212; and enzyme substrates, such as p-nitrophenol acetate radical. Reporter moieties comprising biotin or fluorescent markers are preferred.

Biotin labels can easily be detected or recognized using assays known to one skilled in the art, typically using steptavidin, for example an ALISA assay. Fluorescent labels can also be detected or recognized using assays known to one skilled in the art, for example using flow cytometry. Radioisotopes can also be detected or recognized using assays known to one skilled in the art.

A preferred reporter moiety (M²) comprising biotin is:

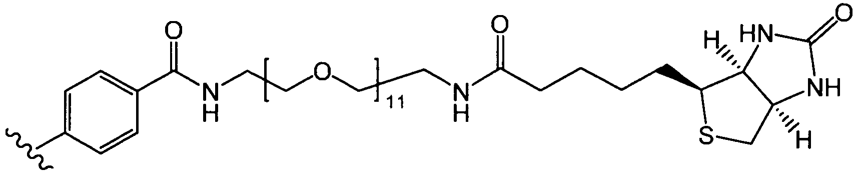

A preferred reporter moiety (M²)are dyes of the Alexa series commercially available at Invitrogen. Thereof a preferred dye is Alexa 488. Several reagents for attachment of Alexa 488 are commercially available at Invitrogen. This dye can easily be detected with its fluorescence, which can be observed at a excitation at 495 nm and an emission at 519 nm. The general structure of Alexa 488 is:

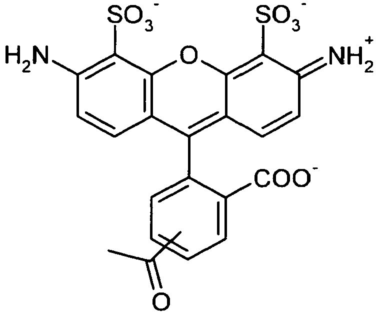

### Dihydroxylamine reagents

The dihydroxylamine compounds used in the present invention are compounds of formula (III):

H₂N-O-B-O-NH₂ (III)

wherein B represents C₂ to C₁₀ alkylene. Said C₂ to C₁₀ alkylene is a linear or branched alkylene, preferably linear. B typically represents a C₂ to C₆ alkylene, preferably a C₂ to C₄ alkylene. B is preferably a n-ethylene, n-propylene or n-butylene group, most preferably an n-propylene group. Thus, a preferred dihydroxylamine compound is 1,3-diaminoxypropane.

The dihydroxylamine compounds of formula (III) are easily prepared from commercially available reagents by techniques known to those skilled in the art, by analogy with known methods.

### Pharmaceutical compositions

The present invention also relates to pharmaceutical compositions comprising a Factor VIII derivative of formula (I). Typically said pharmaceutical composition further comprises a pharmaceutically acceptable carrier or diluent.

A preferred pharmaceutically acceptable carriers or diluents is an aqueous buffered solution. Thus, the present invention relates to a pharmaceutical formulation comprising an aqueous solution of a Factor VIII derivative and a buffer, wherein the Factor VIII derivative is present in a concentration from 0.01 mg/ml or above, and wherein said formulation has a pH from about 2.0 to about 10.0.

Typically, the buffer is selected from the group consisting of sodium acetate, sodium carbonate, citrate, glycylglycine, histidine, glycine, lysine, arginine, sodium dihydrogen phosphate, disodium hydrogen phosphate, sodium phosphate, and tris(hydroxymethyl)-aminomethan, bicine, tricine, malic acid, succinate, maleic acid, fumaric acid, tartaric acid, aspartic acid or mixtures thereof. Each one of these specific buffers constitutes an alternative embodiment of the invention.

Typically, the formulation further comprises a pharmaceutically acceptable preservative. In a further embodiment of the invention the preservative is selected from the group consisting of phenol, o-cresol, m-cresol, p-cresol, methyl p-hydroxybenzoate, propyl p-hydroxybenzoate, 2-phenoxyethanol, butyl p-hydroxybenzoate, 2-phenylethanol, benzyl alcohol, chlorobutanol, and thiomerosal, bronopol, benzoic acid, imidurea, chlorohexidine, sodium dehydroacetate, chlorocresol, ethyl p-hydroxybenzoate, benzethonium chloride, chlorphenesine (3p-chlorphenoxypropane-1,2-diol) or mixtures thereof. In a further embodiment of the invention the preservative is present in a concentration from 0.1 mg/ml to 20 mg/ml. In a further embodiment of the invention the preservative is present in a concentration from 0.1 mg/ml to 5 mg/ml. In a further embodiment of the invention the preservative is present in a concentration from 5 mg/ml to 10 mg/ml. In a further embodiment of the invention the preservative is present in a concentration from 10 mg/ml to 20 mg/ml. Each one of these specific preservatives constitutes an alternative embodiment of the invention. The use of a preservative in pharmaceutical compositions is well-known to the skilled person. For convenience reference is made to Remington: The Science and Practice of Pharmacy, 19th edition, 1995.

Typically, the formulation further comprises an isotonic agent. In a further embodiment of the invention the isotonic agent is selected from the group consisting of a salt (e.g. sodium chloride), a sugar or sugar alcohol, an amino acid (e.g. L-glycine, L-histidine, arginine, lysine, isoleucine, aspartic acid, tryptophan, threonine), an alditol (e.g. glycerol (glycerine), 1,2-propanediol (propyleneglycol), 1,3-propanediol, 1,3-butanediol) polyethyleneglycol (e.g. PEG400), or mixtures thereof. Any sugar such as mono-, di-, or polysaccharides, or water-soluble glucans, including for example fructose, glucose, mannose, sorbose, xylose, maltose, lactose, sucrose, trehalose, dextran, pullulan, dextrin, cyclodextrin, soluble starch, hydroxyethyl starch and carboxymethylcellulose-Na may be used. In one embodiment the sugar additive is sucrose. Sugar alcohol is defined as a C₄-C₈ hydrocarbon having at least one -OH group and includes, for example, mannitol, sorbitol, inositol, galactitol, dulcitol, xylitol, and arabitol. In one embodiment the sugar alcohol additive is mannitol. The sugars or sugar alcohols mentioned above may be used individually or in combination. There is no fixed limit to the amount used, as long as the sugar or sugar alcohol is soluble in the liquid preparation and does not adversely effect the stabilizing effects achieved using the methods of the invention. In one embodiment, the sugar or sugar alcohol concentration is between about 1 mg/ml and about 150 mg/ml. In a further embodiment of the invention the isotonic agent is present in a concentration from 1 mg/ml to 50 mg/ml. In a further embodiment of the invention the isotonic agent is present in a concentration from 1 mg/ml to 7 mg/ml. In a further embodiment of the invention the isotonic agent is present in a concentration from 8 mg/ml to 24 mg/ml. In a further embodiment of the invention the isotonic agent is present in a concentration from 25 mg/ml to 50 mg/ml. Each one of these specific isotonic agents constitutes an alternative embodiment of the invention. The use of an isotonic agent in pharmaceutical compositions is well-known to the skilled person. For convenience reference is made to Remington: The Science and Practice of Pharmacy, 19th edition, 1995.

Typically, the formulation further comprises a chelating agent. In a further embodiment of the invention the chelating agent is selected from salts of ethylenediaminetetraacetic acid (EDTA), citric acid, and aspartic acid, and mixtures thereof. In a further embodiment of the invention the chelating agent is present in a concentration from 0.1 mg/ml to 5 mg/ml. In a further embodiment of the invention the chelating agent is present in a concentration from 0.1 mg/ml to 2 mg/ml. In a further embodiment of the invention the chelating agent is present in a concentration from 2 mg/ml to 5 mg/ml. Each one of these specific chelating agents constitutes an alternative embodiment of the invention. The use of a chelating agent in pharmaceutical compositions is well known to the skilled person. For convenience reference is made to Remington: The Science and Practice of Pharmacy, 19th edition, 1995.

Typically, the formulation further comprises a stabilizer. The use of a stabilizer in pharmaceutical compositions is well known to the skilled person. For convenience reference is made to Remington: The Science and Practice of Pharmacy, 19th edition, 1995.

Typically, the pharmaceutical compositions of the invention may further comprise an amount of an amino acid base sufficient to decrease aggregate formation by the polypeptide during storage of the composition. By "amino acid base" is intended an amino acid or a combination of amino acids, where any given amino acid is present either in its free base form or in its salt form. Where a combination of amino acids is used, all of the amino acids may be present in their free base forms, all may be present in their salt forms, or some may be present in their free base forms while others are present in their salt forms. In one embodiment, amino acids to use in preparing the compositions of the invention are those carrying a charged side chain, such as arginine, lysine, aspartic acid, and glutamic acid. Any stereoisomer (i.e., L, D, or DL isomer) of a particular amino acid (e.g. glycine, methionine, histidine, imidazole, arginine, lysine, isoleucine, aspartic acid, tryptophan, threonine and mixtures thereof) or combinations of these stereoisomers, may be present in the pharmaceutical compositions of the invention so long as the particular amino acid is present either in its free base form or its salt form. In one embodiment the L-stereoisomer is used. Compositions of the invention may also be formulated with analogues of these amino acids. By "amino acid analogue" is intended a derivative of the naturally occurring amino acid that brings about the desired effect of decreasing aggregate formation by the polypeptide during storage of the liquid pharmaceutical compositions of the invention. Suitable arginine analogues include, for example, aminoguanidine, or nithine and N-monoethyl L-arginine, suitable methionine analogues include ethionine and buthionine and suitable cysteine analogues include S-methyl-L cysteine. As with the other amino acids, the amino acid analogues are incorporated into the compositions in either their free base form or their salt form. In a further embodiment of the invention the amino acids or amino acid analogues are used in a concentration, which is sufficient to prevent or delay aggregation of the protein.

In a further embodiment of the invention methionine (or other sulphuric amino acids or amino acid analogous) may be added to inhibit oxidation of methionine residues to methionine sulfoxide when the polypeptide acting as the therapeutic agent is a polypeptide comprising at least one methionine residue susceptible to such oxidation. By "inhibit" is intended minimal accumulation of methionine oxidized species over time. Inhibiting methionine oxidation results in greater retention of the polypeptide in its proper molecular form. Any stereoisomer of methionine (L, D, or DL isomer) or combinations thereof can be used. The amount to be added should be an amount sufficient to inhibit oxidation of the methionine residues such that the amount of methionine sulfoxide is acceptable to regulatory agencies. Typically, this means that the composition contains no more than about 10% to about 30% methionine sulfoxide. Generally, this can be achieved by adding methionine such that the ratio of methionine added to methionine residues ranges from about 1:1 to about 1000:1, such as 10:1 to about 100:1.

Typically, the formulation further comprises a stabilizer selected from the group of high molecular weight polymers or low molecular compounds. In a further embodiment of the invention the stabilizer is selected from polyethylene glycol (e.g. PEG 3350), polyvinyl alcohol (PVA), polyvinylpyrrolidone, carboxy/hydroxycellulose or derivates thereof (e.g. HPC, HPC-SL, HPC-L and HPMC), cyclodextrins, sulphur-containing substances as monothioglycerol, thioglycolic acid and 2-methylthioethanol, and different salts (e.g. sodium chloride). Each one of these specific stabilizers constitutes an alternative embodiment of the invention.

The pharmaceutical compositions may also comprise additional stabilizing agents, which further enhance stability of a therapeutically active polypeptide therein. Stabilizing agents of particular interest to the present invention include, but are not limited to, methionine and EDTA, which protect the polypeptide against methionine oxidation, and a nonionic surfactant, which protects the polypeptide against aggregation associated with freeze-thawing or mechanical shearing.

In a further embodiment of the invention the formulation comprises a surfactant. The surfactant may be a detergent, ethoxylated castor oil, polyglycolyzed glycerides, acetylated monoglycerides, sorbitan fatty acid esters, polyoxypropylene-polyoxyethylene block polymers (eg. poloxamers such as Pluronic® F68, poloxamer 188 and 407, Triton X-100), polyoxyethylene sorbitan fatty acid esters, polyoxyethylene and polyethylene derivatives such as alkylated and alkoxylated derivatives (tweens, e.g. Tween-20, Tween-40, Tween-80 and Brij-35), monoglycerides or ethoxylated derivatives thereof, diglycerides or polyoxyethylene derivatives thereof, alcohols, glycerol, lectins and phospholipids (eg. phosphatidyl serine, phosphatidyl choline, phosphatidyl ethanolamine, phosphatidyl inositol, diphosphatidyl glycerol and sphingomyelin), derivates of phospholipids (eg. dipalmitoyl phosphatidic acid) and lyso-phospholipids (eg. palmitoyl lysophosphatidyl-L-serine and 1-acyl-sn-glycero-3-phosphate esters of ethanolamine, choline, serine or threonine) and alkyl, alkoxyl (alkyl ester), alkoxy (alkyl ether)-derivatives of lysophosphatidyl and phosphatidylcholines, e.g. lauroyl and myristoyl derivatives of lysophosphatidylcholine, dipalmitoylphosphatidylcholine, and modifications of the polar head group, that is cholines, ethanolamines, phosphatidic acid, serines, threonines, glycerol, inositol, and the positively charged DODAC, DOTMA, DCP, BISHOP, lysophosphatidylserine and lysophosphatidylthreonine, and glycerophospholipids (eg. cephalins), glyceroglycolipids (eg. galactopyransoide), sphingoglycolipids (eg. ceramides, gangliosides), dodecylphosphocholine, hen egg lysolecithin, fusidic acid derivatives (e.g. sodium taurodihydrofusidate etc.), long-chain fatty acids and salts thereof C₆-C₁₂ (eg. oleic acid and caprylic acid), acylcarnitines and derivatives, N^{α}-acylated derivatives of lysine, arginine or histidine, or side-chain acylated derivatives of lysine or arginine or histidine, or side-chain acylated derivatives of lysine or arginine, N^{α}-acylated derivatives of dipeptides comprising any combination of lysine, arginine or histidine and a neutral or acidic amino acid, N^{α}-acylated derivative of a tripeptide comprising any combination of a neutral amino acid and two charged amino acids, DSS (docusate sodium, CAS registry no [577-11-7]), docusate calcium, CAS registry no [128-49-4]), docusate potassium, CAS registry no [7491-09-0]), SDS (sodium dodecyl sulphate or sodium lauryl sulphate), sodium caprylate, cholic acid or derivatives thereof, bile acids and salts thereof and glycine or taurine conjugates, ursodeoxycholic acid, sodium cholate, sodium deoxycholate, sodium taurocholate, sodium glycocholate, N-Hexadecyl-N,N-dimethyl-3-ammonio-1-propanesulfonate, anionic (alkyl-aryl-sulphonates) monovalent surfactants, zwitterionic surfactants (e.g. N-alkyl-N,N-dimethylammonio-1-propanesulfonates, 3-cholamido-1-propyldimethylammonio-1-propanesulfonate, cationic surfactants (quaternary ammonium bases) (e.g. cetyltrimethylammonium bromide, cetylpyridinium chloride), non-ionic surfactants (eg. Dodecyl beta-D-glucopyranoside), poloxamines (eg. Tetronic's), which are tetrafunctional block copolymers derived from sequential addition of propylene oxide and ethylene oxide to ethylenediamine, or the surfactant may be selected from the group of imidazoline derivatives, or mixtures thereof. Each one of these specific surfactants constitutes an alternative embodiment of the invention.

The use of a surfactant in pharmaceutical compositions is well-known to the skilled person. For convenience reference is made to Remington: The Science and Practice of Pharmacy, 19th edition, 1995.

It is possible that other ingredients may be present in the pharmaceutical formulation of the present invention. Such additional ingredients may include wetting agents, emulsifiers, antioxidants, bulking agents, tonicity modifiers, chelating agents, metal ions, oleaginous vehicles, proteins (e.g., human serum albumin, gelatine or proteins) and a zwitterion (e.g., an amino acid such as betaine, taurine, arginine, glycine, lysine and histidine). Such additional ingredients, of course, should not adversely affect the overall stability of the pharmaceutical formulation of the present invention.

In an embodiment, the Factor VIII derivative is in dried form, whereto the physician or the patient adds solvents and/or diluents prior to use. By "dried form" is intended the liquid pharmaceutical composition or formulation is dried either by freeze drying (i.e., lyophilization; see, for example, Williams and Polli (1984) J. Parenteral Sci. Technol. 38:48-59), spray drying (see Masters (1991) in Spray-Drying Handbook (5th ed; Longman Scientific and Technical, Essez, U.K.), pp. 491-676; Broadhead et al. (1992) Drug Devel. Ind. Pharm. 18:1169-1206; and Mumenthaler et al. (1994) Pharm. Res. 11:12-20), or air drying (Carpenter and Crowe (1988) Cryobiology 25:459-470; and Roser (1991) Biopharm. 4:47-53).

### Therapeutic utility

The Factor VIII derivatives of the present invention are therapeutically useful, typically in treating the inherited bleeding disorder Haemophilia A (classic haemophilia). Haemophilia A results from a chromosome X-linked deficiency of blood coagulation Factor VIII and affects almost exclusively males with an incidence of between one and two individuals per 10,000. The clinical manifestation of haemophilia A is an increased bleeding tendency.

The Factor VIII derivatives of the invention can therefore be used to alle-viatate the symptoms associated Haemophilia A, or halt further progression or worsening of those symptoms. Typically, treating means a reduction in the tendency of a patient with Haemophilia A to bleed.

The methods comprise administering a therapeutically effective amount of a Factor VIII derivative or pharmaceutical composition of the invention to a patient with Haemophilia A. As used herein, "therapeutically effective amount" includes those amounts that reduce the tendency of a patient with Haemophilia A to bleed. The amount should thus be sufficient to cause a detectable decrease in the severity of the disorder, that is typically a reduction in the tendency to bleed. Preferably the Factor VIII derivatives of the invention are administered as part of a once weekly dosage regime.

The Factor VIII derivatives of the invention may be administered in a variety of dosage forms. Thus, they can be administered parenterally, whether subcutaneously, intravenously, intramuscularly, intrasternally, transdermally or by infusion techniques. The Factor VIII derivatives may also be administered by nasal or pulmonal spray, using a solution or suspension of the Factor VIII derivative in the form of a nasal or pulmonal spray. Transdermal administration includes needleless injection or use of a patch, such as an iontophoretic patch.

A typical dose is from about 15-100 U per kg of body weight, preferably about 20-75 U per kg of body weight, more preferably about 25-50 U per kg of body weight, and most preferably from about 30-40 U per kg of body weight according to the activity of the specific compound, the age, weight and conditions of the subject to be treated, the type and severity of the disease and the frequency and route of administration.

The invention is illustrated by the following Examples:

### EXAMPLES

The following buffer solutions were prepared and used in the preparation of the Intermediates and in the Examples:
- buffer A: 20mM imidazole buffer pH7.3 containing 10mM CaCl₂, 0.02% Tween 80, 1M glycerol and 0.15M NaCl;
- buffer B: 20mM imidazole buffer pH7.3 containing 10mM CaCl₂, 0.02% Tween 80, 1 M glycerol and 0.5M NaCl;
- buffer C: 20mM imidazole buffer pH7.3 containing 10mM CaCl₂, 0.02% Tween 80, 1 M glycerol;
- buffer D: 20mM imidazole buffer pH7.3 containing 10mM CaCl₂, 0.02% Tween 80, 1 M glycerol and 1 M NaCl.
- buffer E: 100mM Imidazole buffer pH 6.5 containing 0.02% Tween 80, 10% v/v glycerol, 10mM CaCl₂;
- buffer F: 5% (w/v) hydroxypropyl β-cyclodextrin; and
- buffer G: 100mM imidazole buffer pH6.5 containing 0.02% Tween 80, 10% v/v glycerol, 0.15M NaCl, 10mM CaCl₂.

### Intermediate 1: 1,3-Diaminoxypropane

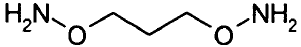

1,8-Diazabicyclo[5,4,0]undec-7-ene (7.9 ml, 53 mmol) was added dropwise to a solution of hydroxyphthalimide (8.68 g, 53 mmol) in N,N-dimethylformamide (50 ml). 1,3-Dibromopropane (2.7 g, 26 mmol) was added. The solution was stirred at 85 °C for 1 hour. It was cooled to room temperature and poured onto ice (200 ml). The mixture was stirred. The formed precipitate was isolated by filtration and was washed with cold water (50 ml) and cold acetonitrile (50 ml). The crude product was recrystallized from butanol (150 ml) and dried to give 2.67 g of 1,3-bis-(1,3-dioxo-1,3-dihydroisoindol-2-yloxy)propane.

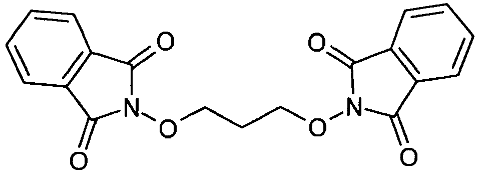

¹H-NMR (CDCl₃): □ 2.22 (quintet, 2 H); 4.51 (t, 4 H); 7.75 (m, 4 H); 7.82 (m, 2 H).

A mixture of 1,3-bis-(1,3-dioxo-1,3-dihydroisoindol-2-yloxy)propane (2.5 g, 6.8 mmol) in concentrated hydrochloric acid (10 ml) and acetic acid (15 ml) was stirred at 115°C for 3 h. The solvents were removed *in vacuo.* Water (15 ml) was added. The precipitation was isolated by filtration. It was washed with 6 M hydrochloric acid (15 ml). The crude product was recrystallized from ethanol to give 480 mg of the hydrochloride salt of 1,3-diaminoxypropane.

¹H-NMR (D₂O): □ 2.05 (quintet, 2 H); 4.13 (t, 4 H).

### Intermediate 2: Transamination of Factor VIII with Intermediate 1 to give N^{Gln}-(3-aminoxy propyloxy) Factor VIII

Microbial transglutaminase (TGase) from *Streptoverticillium mobaraense* (from Ajinomoto) was provided as a powder containing 1% w/w protein. A solution (11.4µM) was made in buffer A.

To the solution of buffer A (11.67ml) was added a solution of a B Domain Deleted Factor VIII compound to which a peptide with the sequence of SFSQNSRHPSQNPPVLKRHQR attached to the C-terminus of the Heavy Chain in buffer B (5.4mg/ml, 400µl), followed by the addition of a solution of 1,3-diaminoxypropane (Intermediate 1) in buffer A (55mg/ml, 6.97ml). The reaction was started by addition of the TGase enzyme solution (11.4µM, 950µl). The reaction mixture was incubated at 27°C for 4 hours. The reaction was stopped by addition of a solution of N-Ethylmaleimide (15.6mg/ml in buffer A, 86µl) and incubated for 10min at 27°C.

The product was purified by ion exchange as follows. The reaction mixture was diluted in buffer C (104ml), and applied on two ion exchange Vivapure Q Maxi M devices (VivaScience product number VS-IX20QM08, Vivascience AG, Germany) which had been previously equilibrated with buffer C. After two washing steps with the same buffer, the reaction product was eluted with the elution buffer D (19ml per device). The resulting eluate was upconcentrated by ultrafiltration on Amicon Ultra devices (50kDa cut-off) (Millipore Corp., USA) down to 1.3ml.

The protein concentration was estimated by measurement of absorption at 280nm (E1%=14.6 Lg-1cm-1)(Nanodrop ND-1000, Nanodrop Technologies, Inc, USA) giving an estimated protein recovery of 97%.

The product (Intermediate 2) was used as such in the subsequent steps.

### Intermediates 3 to 7: preparation of aldehydes

Aldehyde intermediates were prepared using multi-step syntheses as follows below.

### Intermediate 3: 7-((omega-(2-(3-((4-(3-Formylpropyl)-1,2,3-triazol-1-yl)methyl) benzoylamino)ethyl)5 kDa PEGyl)carbamoyl)heptadecanoic acid

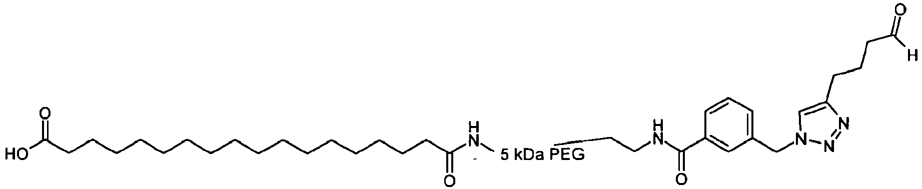

Intermediate 3 contains a PEG group and a -(CH₂)₁₂- albumin binder

### Step 1: methyl 3-(azidomethyl)benzoate

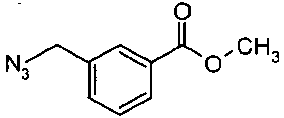

Sodium azide (5.68 g, 87 mmol) was added to a solution of methyl 3-(bromometyl)benzoate (5.00 g, 22 mmol) in N,N-dimethylformamide (50 ml). Tetrabutylammonium iodide (81 mg, 0.22 mmol) was added. The reaction mixture was heated to 60°C for 16 hours. It was cooled to room temperature and given onto water (200 ml). This mixture was extracted with ethyl acetate (400 ml). The organic layer was washed with water (3 x 200 ml) and successively dried over sodium sulphate. The solvent was removed *in vacuo* to give 4.11 g of crude methyl 3-(azidomethyl)benzoate, which was used without further purification.

MS: m/z = 192.

¹H-NMR (CDCl₃): □ 3.92 (s, 3 H); 4.40 (s, 2 H); 7.50 (m, 2 H); 8.00 (m, 2 H).

### Step 2: 3-(Azidomethyl)benzoic acid

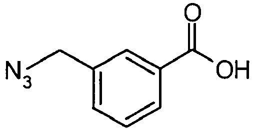

A solution of lithium hydroxide (3.81 g, 21.5 mmol) in water (25 ml) was added to a solution of crude methyl 3-(azidomethyl)benzoate (4.11 g, 21.5 mmol) in 1,4-dioxane (25 ml). Water and 1,4-dioxane was added until a clear solution was obtained. The reaction mixture was stirred for 16 hours at room temperature. A 1M aqueous solution of sodium hydroxide (100 ml) was added. The reaction mixture was washed with tert-butyl methyl ether (2 x 100 ml). The aqueous phase was acidified with a 10% aqueous solution of sodium hydrogensulphate. It was extracted with ethyl acetate (2 x 200 ml). The combined ethyl acetate phases were dried over magnesium sulphate. The solvent was removed in vacuo to give 3.68 g of crude 3-(azidomethyl)benzoic acid, which was used without further purification.

MS: m/z = 150

¹H-NMR (CDCl_{□}□□ 4.57 (s, 3 H); 7.55 (m, 2 H); 8.00 (m, 2 H); 13.10 (br, 1 H).

### Step 3: Pyrrolidin-2,5-dione-1-yl3-(azidomethyl)benozoic ester

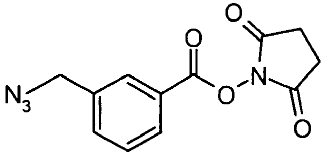

2-Succinimido-1,1,3,3-tetramethyluronium tetrafluoroborate (TSTU, 32.52 g, 107 mmol) was added to a solution of 3-(azidomethyl)benzoic acid (19.01 g, 107 mmol) and triethylamine (14.96 ml, 107 mmol) in *N,N*-dimethylformamide (50 ml). The reaction mixture was stirred for 16 hours at room temperature. It was diluted with ethyl acetate (250 ml) and washed with water (3 x 120 ml). The organic layer was washed with a saturated aqueous solution of sodium hydrogencarbonate (150 ml) and dried over sodium sulphate. The solvent was removed *in vacuo* to give 25.22 g of pyrrolidin-2,5-dione-1-yl 3-(azidomethyl)benozoic ester.

¹H-NMR (CDCl₃) □ 2.92 (m, 4 H); 4,45 (s, 2 H); 7.55 (t, 1 H), 7.65 (d, 2 H); 8.10 (m, 2 H).

### Step 4: octadecanedioic acid mono-tert-butyl ester

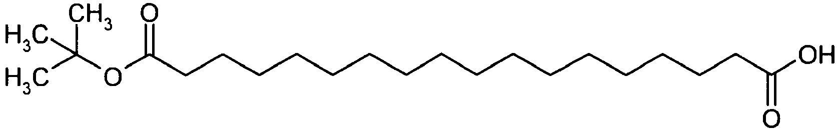

N,N-Dimethylformamide di-tert-butylacetal (35.2 ml, 147 mmol) was added dropwise to a solution of octadecanedioic acid (15.4 g, 49.0 mmol) in toluene (250 ml) which was kept at 95 °C. The reaction mixture was kept at 95 °C for 16 hours. It was cooled to room temperature. The solvent was removed *in vacuo*. The residue was dissolved in dichloromethane (150 ml). The solvent was removed *in vacuo.* The residue was dissolved in dichloromethane (150 ml). The solvent was removed *in vacuo.* The residue was dissolved in dichloromethane (150 ml). The solvent was removed *in vacuo.* The residue was dissolved in dichloromethane (85 ml). The insoluble material was removed by filtration. The solvent was removed *in vacuo* from the filtrate. The residue was dissolved in dichloromethane (18 ml). Heptane (180 ml) was added. The formed precipitation was removed by filtration. The solvent was removed *in vacuo.* Heptane (150 ml) was added. A precipitation was formed. This was isolated by filtration and dried *in vacuo*. The solvent was removed *in vacuo* from the mother liquor. The formed solid was isolated by filtration and dried *in vacuo.* The two batches of isolated solids were combined and dissolved in the smallest possible amount of refluxing dichloromethane. The solution was cooled to room temperature. Heptane (10 times the volume of dichloromethane) was added. The mixture was kept at 0 °C. The formed precipitation was isolated by filtration, washed with heptane (30 ml) and dried *in vacuo to* give 4.19 g of octadecanedioic acid mono-tert-butyl ester.

¹H-NMR (DMSO-d₆) □ 1.23 (m, 24 H); 1.39 (s, 9 H); 1.47 (m, 4 H); 2.16 (m, 4 H).

### Step 5: Octadecanedioic acid tert-butyl ester 2,5-dioxopyrrolidin-1-yl ester

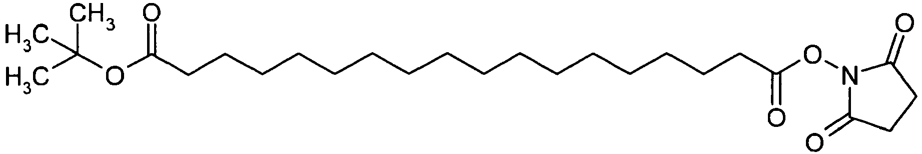

2-Succinimido-1,1,3,3-tetramethyluronium tetrafluoroborate (TSTU, 378 mg, 0.71 mmol) was added to a solution of octadecanedioic acid mono-tert-butyl ester (300 mg, 0.64 mmol) in dichloromethane (10 ml). Ethyldiisopropylamine (0.152 ml, 0.71 mmol) was added to the reaction mixture. It was stirred at room temperature for 3 days. It was washed with a 10% aqueous solution of sodium hydrogensulphate (2 x 10 ml), with a saturated solution of sodium hydrogencarbonate (10 ml), and finally with brine (10 ml). The organic layer was dried over magnesium sulphate. The solvent was removed in vacuo to give octadecanedioic acid tert-butyl ester 2,5-dioxopyrrolidin-1-yl ester, which was used without further purification.

¹H-NMR (CDCl₃) □ 1.20-1.65 (m, 26 H); 1.44 (s, 9 H); 1.74 (quintet, 2 H); 2.20 (t, 2 H); 2.60 (t, 2 H); 2.84 (m, 4 H).

### Step 6: Hex-5-ynal

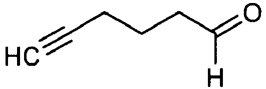

A solution of dimethylsulphoxide (0.13 ml, 2 mmol) was cooled to -78°C. A solution of oxyalyl chloride (0.12 ml, 1.38 mmol) in dichloromethane (1 ml) was added to this solution. The mixture was stirred at -78°C for 20 min. A solution of hex-5-yn-1-ol (0.10 ml, 0.922 mmol) in dichloromethane (0.5 ml) was added to this solution. It was stirred for 20 min at -78 °C. Triethylamine (0.51 ml, 3.69 mmol) was added. The reaction mixture was stirred at -78 °C for 10 min. The solution was allowed to warm to room temperature. It was diluted with ethyl acetate (40 ml) and washed with a 10% aqueous solution of sodium hydrogensulphate (2 x 20 ml). The organic layer was washed with brine (20 ml) and dried over magnesium sulphate. The solvent was removed *in vacuo* to give hex-5-ynal.

¹H-NMR (CDCl₃) □ 1.86 (quintet, 2 H); 2.27 (t, 2 H); 2.62 (t, 2 H), 9.81 (s, 1 H).

### Step 7: 3-(Azidomethyl)-N-(omega-(2-(tert-Butoxycarbonylamino)ethyl) 5 kDa PE-Gyl)benzoic amide

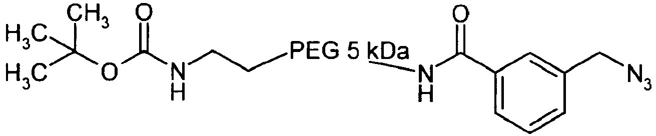

Pyrrolidin-2,5-dione-1-yl 3-(azidomethyl)benozoic ester (64 mg, 0.234 mmol) and ethyldiisopropylamine (0.10 ml, 0.585 mmol) were added subsequently to a solution of commercially available tert-butyl 2-(omega-(amino)5 kDa PEGyl)ethylcarbamate (e.g. Rapp, 1.00 g, 0.195 mmol). The reaction mixture was stirred for 16 h at room temperature. The solvent was removed *in vacuo.* Ether (20 ml) was added. The formed precipitation was isolated by filtration and dried *in vacuo* to give 3-(azidomethyl)-N-(omega-(2-(tert-Butoxycarbonylamino)ethyl) 5 kDa PEGyl)benzoic amide. The ¹H-NMR in CDCl₃ met the expectation.

### Step 8: tert-Butyl 17-(2-(omega-(3-(azidomethyl)benzoylamino)5 kDa PE-Gyl)ethylcarbamoyl)heptadecanoate

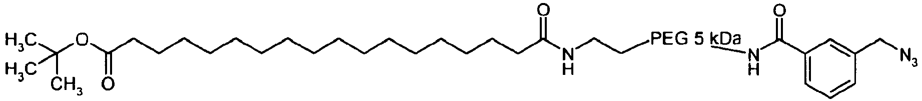

Trifluoroacetic acid (5 ml) was added to a solution of 3-(azidomethyl)-N-(omega-(2-(tert-butoxycarbonylamino)ethyl) 5 kDa PEGyl)benzoic amide (1.03 g, 0.19 mmol) in dichloromethane (5 ml). The reaction mixture was stirred for 15 min at room temperature. The solvent was removed *in vacuo.* The residue was dissolved in dichloromethane (10 ml). The solvent was removed *in vacuo.* The residue was dissolved in dichloromethane (10 ml). The solvent was removed *in vacuo.* The residue was dissolved in dichloromethane (10 ml). The solvent was removed *in vacuo.* The residue was dissolved in dichloromethane (10 ml). A solution of octadecanedioic acid tert-butyl ester 2,5-dioxopyrrolidin-1-yl ester (90 mg, 0.243 mmol) in dichloromethane (5 ml) and ethyldiisopropylamine (0.83 ml, 4.86 mmol) were added subsequently. The reaction mixture was stirred for 16 h at room temperature. It was diluted with dichloromethane (40 ml) and washed with a 10% aqueous solution of sodium hydrogensulphate (2 x 30 ml) and brine (30 ml). The solution was dried over magnesium sulphate. The solvent was removed *in vacuo.* The remaining oil was treated with ether (30 ml). The formed solid was isolated by filtration. It was washed with ether (10 ml) and dried *in vacuo* to give 783 mg of tert-butyl 17-(2-(omega-(3-(azidomethyl)benzoylamino)5 kDa PE-Gyl)ethylcarbamoyl)heptadecanoate. The ¹H-NMR in CDCl₃ met the expectation.

### Step 9: 17-(2-(omega-(3-(Azidomethyl)benzoylamino)5 kDa PE-Gyl)ethylcarbamoyl)heptadecanoic acid

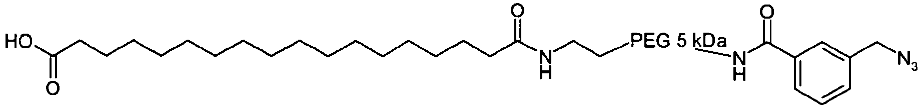

Trifluoroacetic acid (5 ml) was added to a solution of tert-butyl 17-(2-(omega-(3-(azidomethyl)benzoylamino)5 kDa PEGyl)ethylcarbamoyl)heptadecanoate (500 mg, 0.09 mmol) in dichloromethane (5 ml). The reaction mixture was stirred for 45 min at room temperature. The solvent was removed in vacuo. The residue was dissolved in dichloromethane (25 ml). It was washed subsequently with a satd. aqueous solution of sodium hydrogencarbonate (25 ml) and brine (25 ml). The organic layer was dried over magnesium sulphate. The solvent was removed in vauco to give 17-(2-(omega-(3-(azidomethyl)benzoylamino)5 kDa PEGyl)ethylcarbamoyl)heptadecanoic acid.

### Step 10: 17-((omega-(2-(3-((4-(3-Formylpropyl)-1,2,3-triazol-1-yl)methyl)benzoylamino)ethyl)5 kDa PEGyl)carbamoyl)heptadecanoic acid(Intermediate 3)

17-(2-(omega-(3-(Azidomethyl)benzoylamino)5 kDa PE-Gyl)ethylcarbamoyl)heptadecanoic acid (200 mg, 0.036 mmol) was dissolved in a buffer consisting of 2% 2,6-lutidine in water (3.5 ml). A solution of hex-5-ynal (70 mg, 0.725 mmol) in ethanol (0.5 ml) was added. A solution of copper (II) sulphate pentahydrate (180 mg) in water (2.50 ml) was added to a solution of ascorbic acid (638 mg, 3.626 mmol) in a mixture of water (2.5 ml) and 2,6-lutidine (0.125 ml). This solution was kept for 45 seconds at room temperature before it was added to the solution of 17-(2-(omega-(3-(azidomethyl)benzoylamino)5 kDa PEGyl)ethylcarbamoyl)heptadecanoic acid and hexynal. The reaction mixture was stirred at room temperature for 16 hours. It was diluted with water (25 ml) and extracted with dichloromethane (2 x 100 ml). The dichloromethane phase was washed with a 10% aqueous solution of sodium hydrogensulphate (2 x 100 ml) and brine (100 ml). It was dried over magnesium sulphate. The solvent was removed *in vacuo*. Ether (30 ml) was added. The formed precipitation was isolated by filtration. It was redissolved in a 50 mM aqueous solution of sodium hydrogencarbonate (5 ml) filtered and subjected to a gel chromatography using a HiPrep 26/10 Desalting column (GE Healthcare) and a buffer of 50 mM ammonium hydrogencarbonate. The fractions containing the desired material were pooled and freeze dried to give 17-((omega-(2-(3-((4-(3-formylpropyl)-1,2,3-triazol-1-yl)methyl)benzoylamino)ethyl)5 kDa PEGyl)carbamoyl)heptadecanoic acid (Intermediate 3).

The ¹H-NMR analysis showed approximately 10% of the expected aldehyde (Intermediate 3).

### Intermediate 4: 19-(((trans-4-((S)-3-((S)-1-(2-(2-((2-(2-(1-(formylmethylcarbamoyl)-methylcarbamoyl)methoxy)ethoxy)ethylcarbamoyl)methoxy)ethoxy)ethylcarbamoyl)-3-carboxypropylcarbamoyl)-1-carboxypropylcarbamoyl)-cyclohexyl)methyl)carbamoyl)nonadecanoic acid

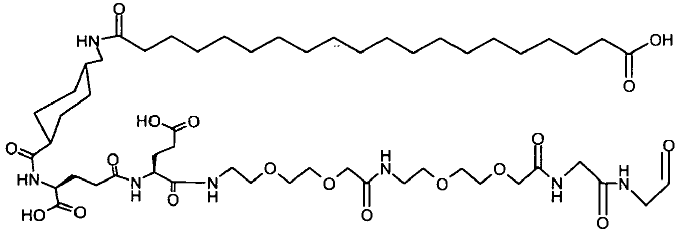

### Step 1: icosanedioic acid mono-tert-butyl ester

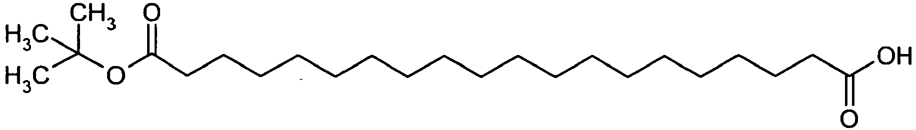

Icosanedioic acid (10 g, 29 mmol) was dissolved in toluene at 115°C. The solution was kept at this temperature, while N,N-dimethylformamide di-tert-butylacetal was added dropwise over 1 h. The reaction mixture was stirred at 115°C for 16 h. It was cooled to 0 °C. The formed precipitation was removed by filtration. The solvent was removed from the filtrate to give 7.49 g of icosanedioic acid mono-tert-butyl ester.

¹H-NMR (CDCl₃): □ 1.25 (m,28 H); 1.44 (s, 9 H); 1.59 (m, 4 H); 2.20 (t, 2 H); 2.34 (t, 2 H).

### Step 2: 19-(((trans-4-((S)-3-((S)-1-(2-(2-((2-(2-(((2,2-di-methoxyethylcarbamoyl)-methylcarbamoyl)methoxy)ethoxy)ethylcarbamoyl)methoxy)ethoxy)ethylcarbamoyl)-3-carboxypropylcarbamoyl)-1-carboxypropylcarbamoyl)cyclohexyl)methyl)carbamoyl) non-adecanoic acid

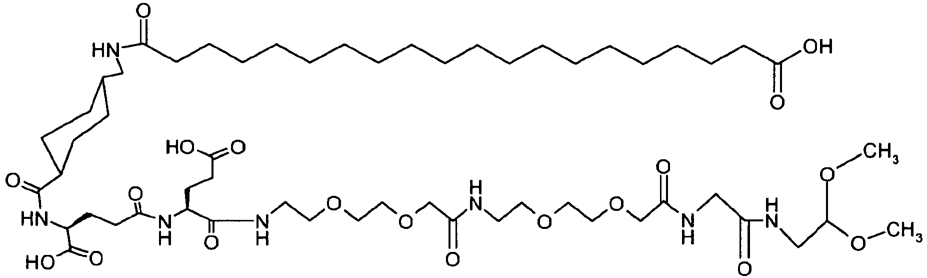

A commercially available resin Boc-Gly-PAM resin (e.g. Fluka, 0.25 mmol) was treated briefly with trifuoroacetic acid (10 ml). The solvent was removed and the resin was washed with N,N-dimethylformamide (3 x 10 ml). The resin was transferred onto a ABI433 peptide synthesizer. For the following couplings standard programs were used, using 1-hydroxybenzotriazole/2-(1H-benzotriazol-1-yl)-1,1,3,3,-tetramethylurionium hexafluorophosphate as coupling reagent. The following acids (each of them 1 mmol) were used in the order of:
- FmocNH-CH₂-CH₂-O-CH₂-CH₂-O-CH₂-COOH
- FmocNH-CH₂-CH₂-O-CH₂-CH₂-O-CH₂-COOH
- Fmoc-Glu(OtBu)-OH
- Fmoc-Glu(OH)-OtBu
- Fmoc-Tranexamic acid
- Icosanedioic acid mono-tert-butyl ester

The resin was subsequently treated with a mixture of trifluoroacetic acid (2 ml), triisopropysilane (0.050 ml) and water (0.050 ml) for 1 h. The solvent was removed. The resin was treated at 45 °C with a mixture of chloroform and aminoacetaldehydedimethyl acetal in a ratio of 3:2 (2 ml) for 20 h. The resin was removed by filtration. The solvent was removed from the filtrate *in vacuo.* The residue was dissolved in a 50% solution of acetonitrile in water. A few drops of acetic acid was added until a slightly acidic solution was obtained. The peptide was purified by HPLC using a gradient of 40-60% acetonitrile in water as eluent, wherein both solvents were buffered with 0.1% trifluoroacetic acid. The peptide was finally isolated by freeze-drying.

### Step 3: 19-(((trans-4-((S)-3-((S)-1-(2-(2-((2-(2-(1-(formylmethylcarbamoyl)ethyl car bamoyl)methoxy)ethoxy)ethylcarbamoyl)methoxy)ethoxy)ethylcarbamoyl)-3-carboxypropylcarbamoyl)-1-carboxypropylcarbamoyl)-cyclohexyl)methyl)carbamoyl)nonadecanoic acid

The aldehyde was obtained by treatment with TFA according to Lelièvre et al. Tetr. Lett. 39 (1998), 9675. Briefly, the dimethylacetal [19-(((trans-4-((S)-3-((S)-1-(2-(2-((2-(2-(((2,2-di-methoxyethylcarbamoyl)methylcarbamoyl)methoxy)ethoxy)ethylcarbamoyl)-methoxy)ethoxy)ethylcarbamoyl)-3-carboxypropylcarbamoyl)-1-carboxypropylcarbamoyl)-cyclohexyl)methyl)carbamoyl)nonadecanoic acid] (2mg) was treated with TFA (50µl) for 6min at ambient temperature, and evaporated to dryness under vacuum. Remaining TFA was stripped off by adding EtOH (50µl) and evaporating under vacuum (repeated three times). The product obtained was dissolved in 5% (w/v) hydroxypropyl β-Cyclodextrin and used immediately in the coupling to *N*^{Gln}-(3-(aminoxy)propyloxy) FVIII (Intermediate 2).

### Intermediate 5: N-(2-(2-(2-(2-(2-(2-(2-(2-(2-(2-(2-(2-(Biotinylamino)ethoxy)ethoxy)-ethxoy)ethoxy)ethoxy)ethoxy)ethoxy)ethoxy)ethoxy)ethoxy)ethoxy)ethyl)-4-formylbenzoic amide

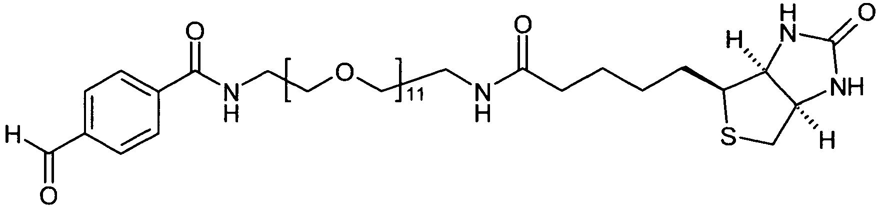

Intermediate 5 contains a biotin reporter moiety.

### Step 1: 4-Formylbenzoic acid 2,5-dioxopyrrolidin-1-yl ester

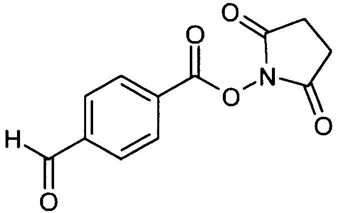

Triethylamine (2.04 ml, 14.65 mmol) and 2-succinimido-1,1,3,3-tetramethyluronium tetrafluoroborate (TSTU, 4.44 g, 14.65 mmol) were successively added to a solution of 4-formylbenzoic acid (2.0 g, 13.3 mmol) in N,N-dimethylformamide (30 ml). The reaction mixture was stirred at room temperature for 16 h. It was diluted with ethyl acetate (150 ml) and washed with a 10% aqueous solution of sodium hydrogen sulphate (100 ml). The aqueous phase was extracted with ethyl acetate (2 x 30 ml). The combined organic layers were washed with a mixture of brine (50 ml) and water (50 ml). The combined organic layers were dried over magnesium sulphate. The solvent was removed in vacuo. The crude product was recrystallized from ethyl acetate to give 1.89 g of 4-formylbenzoic acid 2,5-dioxopyrrolidin-1-yl ester.

¹H-NMR (CDCl₃). □ 2.95 (s, 4 H); 8.04 (d, 2 H), 8.32 (d, 2 H); 10.15 (s, 1 H).

### Step 2: N-(2-(2-(2-(2-(2-(2-(2-(2-(2-(2-(2-(2-(tert-Butoxycarbonylamino)ethoxy)-ethoxy)ethxoy)ethoxy)ethoxy)ethoxy)ethoxy)ethoxy)ethoxy)ethoxy)ethoxy)ethyl)-4-formylbenzoic amide

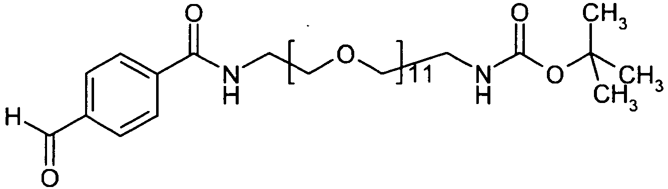

4-Formylbenzoic acid 2,5-dioxopyrrolidin-1-yl ester (0.767 g, 3.11 mmol) was added to a solution of commercially available tert-butyl 2-(2-(2-(2-(2-(2-(2-(2-(2-(2-(2-(2-aminoethoxy)ethoxy)ethoxy)ethoxy)ethoxy)ethoxy)ethoxy)ethoxy)-ethoxy)ethoxy)ethoxy)ethylcarbamate (eg. Fluka, 2.0 g, 3.11 mmol) and ethyldiisopropylamine (0.64 ml, 3.72 mmol) in dichloromethane (30 ml). The reaction mixture was stirred at room temperature for 16 h. The reaction mixture was diluted with dichloromethane (70 ml). It was washed with a 10% aqueous solution of sodium hydrogensulphate (100 ml). The aqueous phase was extracted with dichloromethane (2 x 30 ml). the combined organic layers were washed with a saturated aqueous solution of sodium hydrogencarbonate (70 ml). They were dried over magnesium sulphate. The solvent was removed in vacuo to give 2.15 g of crude *N*-(2-(2-(2-(2-(2-(2-(2-(2-(2-(2-(2-(2-(tert-butoxycarbonylamino)ethoxy)ethoxy)ethxoy)-ethoxy)ethoxy)ethoxy)ethoxy)ethoxy)ethoxy)ethoxy)ethoxy)ethyl)-4-formylbenzoic amide, which was used in the following step without further purification.

¹H-NMR (CDCl₃). □ 1.46 (s, 9 H); 3.33 (m, 2 H); 3.54-3.75 (m, 46 H); 5.08 (br, 1 H); 7.20 (br, 1 H); 7.97 (d, 2 H); 8.03 (d, 2 H); 10.10 (s, 1 H).

MS: m/z = 799, 677 required for [M+Na]⁺: 799, required for [M+1-Boc]⁺: 677

### Step 3 N-(2-(2-(2-(2-(2-(2-(2-(2-(2-(2-(2-(2-(Amino)ethoxy)ethoxy)ethxoy)ethoxy)-ethoxy)ethoxy)ethoxy)ethoxy)ethoxy)ethoxy)ethoxy)ethyl)-4-formylbenzoic amide

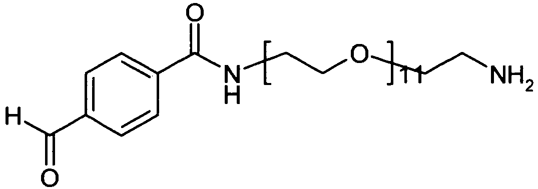

Trifluoroacetic acid (15 ml) was added to a solution of crude *N*-(2-(2-(2-(2-(2-(2-(2-(2-(2-(2-(2-(2-(tert-butoxycarbonylamino)ethoxy)ethoxy)ethxoy)ethoxy)ethoxy)ethoxy)-ethoxy)ethoxy)ethoxy)ethoxy)ethoxy)ethyl)-4-formylbenzoic amide (2.15 g, 2.77 mmol) in dichloromethane (15 ml). The reaction mixture was stirred at room temperature for 75 min. The solvent was removed in vacuo. The residue was dissolved in dichloromethane (30 ml). The solvent was removed in vacuo. The residue was dissolved in dichloromethane (30 ml). The solvent was removed in vacuo. The residue was dissolved in dichloromethane (30 ml). The solvent was removed in vacuo. The obtained crude product was purifie on HPLC, using a reversed phase C18 column and a gradient fo 11-49% acetonitrile in water, wherein both solvents were buffered by 0.1% trifluoroacetic acid. The fractions containing the desired compound were combined and freeze dried to give 995 mg of the trifluoroacetic acid salt of *N*-(2-(2-(2-(2-(2-(2-(2-(2-(2-(2-(2-(2-(amino)ethoxy)ethoxy)ethxoy)ethoxy)ethoxy)ethoxy)-ethoxy)ethoxy)ethoxy)ethoxy)ethoxy)ethyl)-4-formylbenzoic amide.

¹H-NMR (CDCl₃). □ 3.18 (m, 2 H), 3.50-380 (m, 46 H); 7.42 (br, 1 H); 7.61 (br, 3 H); 7.97 (d, 2 H); 8.02 (d, 2 H); 10.08 (s, 1 H).

MS: m/z = 677, 699, required for [M+1]⁺: 677, required for [M+Na]⁺: 699.

### Step 4: N-(2-(2-(2-(2-(2-(2-(2-(2-(2-(2-(2-(2-(Biotinylamino)ethoxy)ethoxy)ethxoy)-ethoxy)ethoxy)ethoxy)ethoxy)ethoxy)ethoxy)ethoxy)ethoxy)ethyl)-4-formylbenzoic amide

Commercially available succinimido biotin (e.g. Fluka, 302 mg, 0.886 mmol) was added to a solution of the trifluoroacetic acid salt of *N*-(2-(2-(2-(2-(2-(2-(2-(2-(2-(2-(2-(2-(amino)ethoxy)ethoxy)ethxoy)ethoxy)ethoxy)ethoxy)ethoxy)-ethoxy)ethoxy)ethoxy)ethoxy)ethyl)-4-formylbenzoic amide (545 mg, 0.805 mmol) and ethyldiisopropylamine (2.10 ml, 12.1 mmol) in dichloromethane (10 ml). The reaction mixture was stirred for 3 days at room temperature. The solvent was removed in vacuo. The crude product was purified by HPLC, using a reversed phase C18 column and a gradient of 15-50% acetonitrile in water, wherein both solvents were buffered with 0.1 % trifuloroacetic acid. The fractions containing the desired product in a reasonable purity - as judged by analytical HPLC - were combined and freeze-dried to give 62 mg of *N*-(2-(2-(2-(2-(2-(2-(2-(2-(2-(2-(2-(2-(biotinylamino)ethoxy)ethoxy)ethxoy)ethoxy)ethoxy)ethoxy)ethoxy)-ethoxy)ethoxy)ethoxy)ethoxy)ethyl)-4-formylbenzoic amide.

¹H-NMR (CDCl₃). □ 1.47 (m, 2 H); 1.70 (m, 4 H); 2.25 (hidden under water (?), 2 H); 2.78 (d, 1 H); 2.93 (d, 1 H); 3.19 (d, 1 H); 3.40-3.75 (m, 46 H); 4.38 (d, 1 H); 4.56 (d, 1 H); 5.55 (br, 1 H); 6.03 (br, 1 H); 6.59 (br, 1 H); 7.31 (br, 1 H); 7.95 (d, 2 H); 8.02 (d, 2 H), 10.08 (s, 1 H).

MS: m/z = 903, required for [M+1]⁺: 903.

### Intermediate 6: N-2-(2-(2-(2-(2-(2-(2-(2-(2-(2-(2-(2-( 4-Formylbenzoylamino)ethoxy)-ethoxy)ethoxy)ethoxy)ethoxy)ethoxy)ethoxy)ethoxy)ethoxy)ethoxy)ethoxy)ethylAlexa 488 carboxylic amide

A solution of commercially available Alexa 488 carboxylic acid succinimidyl ester (Invitorogen, A20000, 1 mg, 0.002 mmol) in a 250 mM aqueous solution of sodium hydrogencarbonate (0.50 ml) was added to a solution of N-(2-(2-(2-(2-(2-(2-(2-(2-(2-(2-(2-(2-(Amino)ethoxy)ethoxy)ethxoy)ethoxy)ethoxy)ethoxy)ethoxy)-ethoxy)ethoxy)ethoxy)ethoxy)ethyl)-4-formylbenzoic amide (1.89 mg, 0.003 mmol) in a 250 mM aqueous solution of sodium hydrogencarbonate (0.482 ml). The reaction mixture was stirred at room temperature for 3 days. A 10% aqueous solution of sodium hydrogensulphate (5 ml) was added. The mixture was subjected to a HPLC chromatography on a C18-reversed phase column, using a gradient of 15-45% of a 0.1 % solution of trifluoroacetic acid in acetonitrile in a 0.1% solution of trifluoroacetic acid in water. The fractions containing the desired compound - according to LC-MS - were combined and lyophilized.

LC-MS: found: m/z = 1193, required for [M+1]⁺: m/z = 1193.

### Intermediate 7: S³⁴-(1-(2-(2-(2-(2-(2-(2-(2-(2-(2-(2-(2-(2-(2-(2-(3-((S)-2-amino-3-hydroxypropanoyl amino)propylcarbamoyl)ethoxy)ethoxy)ethoxy)ethoxy)ethoxy) ethoxy)ethoxy)ethoxy)ethoxy)ethoxy)ethoxy)ethoxy)ethylcarbamoyl)ethyl)2,5-dioxo-2,5-dihydropyrrol-3-yl)albumin

### Step 1: [3-((S)-3-(tert-butoxy)-2-(tert-butoxycarbonylamino)propionylamino)propyl] carbamic acid benzyl ester

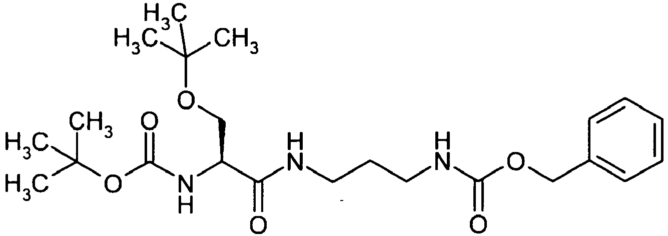

Commercially available Boc-Ser(tBu)-OH (1.14 g, 4.36 mmol) was dissolved in a mixture of dichloromethane (10 ml) and N,N-dimethylformamide (10 ml). 1-Hydroxybenzotriazole (0.71 g, 5.23 mmol) was and 1-(3-dimethylaminopropyl)-3-ethylcarbodiimide hydrochloride (0.92 g, 4.79 mmol) were added successively. The reaction mixture was stirred for 15 min at room temperature. Commercially available (3-aminopropyl)carbamic acid benzyl ester (2.13 g, 8.71 mmol) and ethyldiisopropylamine (3.73 ml, 21.77 mmol) were added.

The reaction mixture was stirred for 6 days at room temperature. It was diluted with dichloromethane (50 ml), washed with a 10% aqueous solution of sodium hydrogensulphate (2 x 50 ml), with a saturated aqueous solution of sodium hydrogencarbonate (2 x 50 ml) and finally with brine (50 ml). It was dried over magnesium sulphate. The solvent was removed in vacuo to give 2.3 g of crude [3-((S)-3-(tert-butoxy)-2-(tert-butoxycarbonylamino)propionylamino)propyl]carbamic acid benzyl ester, which was used without further purification in the next step.

MS: found: m/z = 474, required for [M+Na]⁺: 474

¹H-NMR (CDC13): □ 1.16 (s, 9 H); 1.46 (s, 9 H); 1.64 (m, 2 H); 3.22 (m, 2 H); 3.39 (m, 2 H); 3.80 (m, 1 H); 4.16 (br, 1 H); 5.10 (s, 2 H); 5.42 (br, 1 H); 5.46 (br, 1 H); 6.76 (br, 1 H); 7.35 (m, 5 H).

### Step 2: [(S)-1-(3-Aminopropylcarbamoyl)-2-(tert-butoxy)ethyl]carbamic acid tert-butyl ester

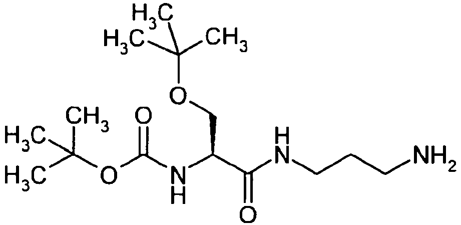

Crude [3-((S)-3-(tert-butoxy)-2-(tert-butoxycarbonylamino)propionylamino) propyl]carbamic acid benzyl ester (2.3 g) from Step 1 was dissolved in ethanol (20 ml). 5% palladium in charcoal, which was 50% wet (1 g) was added. The mixture was hydrogenated at room temperature at a pressure of 100 psi. The reaction mixture was filtered through a plug of celite. The solvent was removed in vacuo from the filtrate. The residue was redissolved in ethanol (20 ml). 5% palladium in charcoal, which was 50% wet (1.7 g) was added.

The mixture was hydrogenated at room temperature at a pressure of 300 psi. It was filtered through a plug of celite. The solvent was removed in vacuo from the filtrate to give crude [(S)-1-(3-aminopropylcarbamoyl)-2-(tert-butoxy)ethyl]carbamic acid tert-butyl ester (1.35 g), which was used in the next step withouth further purification.

¹H-NMR (CDCl₃):□ 1.17 (s, 9 H); 1.46 (s, 9 H); 2.05 (br, 2 H); 2.85 (br, 2 H); 3.49 (br, 2 H); 3.75 (m, 1 H); 4.22 (br, 1 H); 5.55 (br, 1 H); 7.47 (br, 1 H); 8.38 (br, 2 H).

### Step 3: (S)-3-(tert-Butoxy)-2-(tert-butoxycarbonylamino)-N-(3-((2-(2-(2-(2-(2-(2-(2-(2-(2-(2-(2-(2-(2-(3-(2,5-Dioxo-2,5-dihydropyrrol-1-yl)propionylamino)ethoxy)ethoxy) ethoxy)ethoxy)ethoxy)ethoxy)ethoxy)ethoxy)ethoxy)ethoxy)ethoxy)ethoxy)ethyl) carbonylamino)propyl)propoinic amide

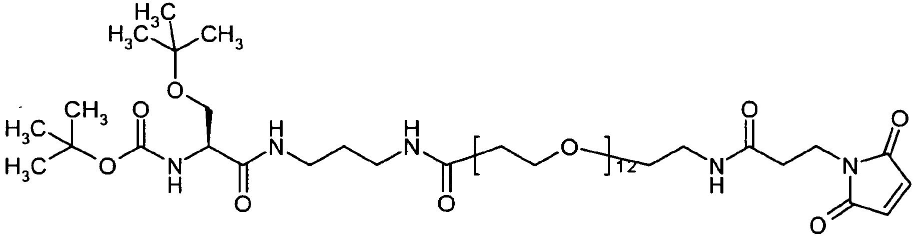

Crude [(S)-1-(3-aminopropylcarbamoyl)-2-(tert-butoxy)ethyl]carbamic acid tert-butyl ester (650 mg, 2.05 mmol), from the previous step, was dissolved in dichloromethane (20 ml). Commercially available (e.g. QunataBioDesign N-succinidyl 3-(2-(2-(2-(2-(2-(2-(2-(2-(2-(2-(2-(2-(3-(2,5-dioxo-2,5-dihydro-pyrrol-1-yl)propionylamino)ethoxy)ethoxy)ethoxy)ethoxy)-ethoxy)ethoxy)ethoxy)ethoxy)ethoxy)ethoxy)ethoxy)ethoxy) propionate) (1.20 g, 1.39 mmol) and ethyldiisopropylamine (0.45 ml, 2.63 mmol) were added successively.

The reaction mixture was stirred for 1.5 h at room temperature. It was washed with a 10% aqueous solution of sodium hydrogensulphate (2 x 30 ml), a saturated aqueous solution of sodium hydrogencarbonate (30 ml) and brine (30 ml). The organic layer was dried over magnesium sulphate. The solvent was removed in vacuo to give 1.2 g of crude (S)-3-(tert-butoxy)-2-(tert-butoxycarbonylamino)-N-(3-((2-(2-(2-(2-(2-(2-(2-(2-(2-(2-(2-(2-(2-(3-(2,5-Dioxo-2,5-dihydro-pyrrol-1-yl)propionylamino)ethoxy)ethoxy)ethoxy)ethoxy)ethoxy)ethoxy)-ethoxy)ethoxy)ethoxy)ethoxy)ethoxy)ethoxy)ethyl)carbonylamino) propyl)propoinic amide, which was used in the next step without further purification.

MS: m/z = 1068, required for [M+1]⁺: 1068.

### Step 4: (S)-2-Amino-N-(3-((2-(2-(2-(2-(2-(2-(2-(2-(2-(2-(2-(2-(2-(3-(2,5-Dioxo-2,5-dihydropyrrol-1-yl)propionylamino)ethoxy)ethoxy)ethoxy)ethoxy)ethoxy)ethoxy)ethoxy)-ethoxy)ethoxy)ethoxy)ethoxy)ethoxy)ethyl)carbonylamino)propyl)-3-hydroxypropoinic amide

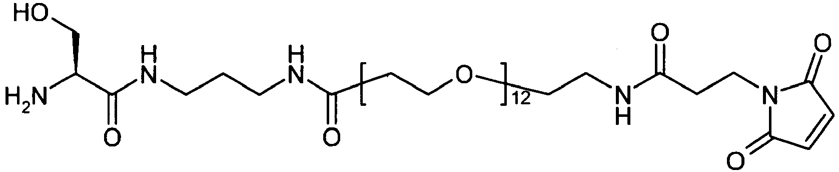

TFA (5 ml) was added to a solution of (S)-3-(tert-butoxy)-2-(tert-butoxycarbonylamino)-*N*-(3-((2-(2-(2-(2-(2-(2-(2-(2-(2-(2-(2-(2-(2-(3-(2,5-Dioxo-2,5-dihydropyrrol-1-yl)propionylamino)ethoxy)ethoxy)ethoxy)ethoxy)ethoxy)ethoxy)ethoxy)-ethoxy)ethoxy)ethoxy)ethoxy)ethoxy)ethyl)carbonylamino)propyl)propoinic amide (600 mg, 0.56 mmol) in dichloromethane (5 ml). The mixture was stirred at room temperature for 1.5 h. The solvent was removed in vacuo. The residue was redissolved in dichloromethane (20 ml). The solvent was removed in vacuo. The latter prodedure was repeated. The residue was dissolved in acetonitrile (20 ml). The solvent was removed in vacuo.

The latter procedure was repeated twice to give (S)-2-amino-*N*-(3-((2-(2-(2-(2-(2-(2-(2-(2-(2-(2-(2-(2-(2-(3-(2,5-Dioxo-2,5-dihydro-pyrrol-1-yl)propionylamino)ethoxy)ethoxy) ethoxy)ethoxy)ethoxy)ethoxy)ethoxy)ethoxy)ethoxy)ethoxy)ethoxy)ethoxy)ethyl) carbonylamino)propyl)-3-hydroxypropoinic amide.

MS: m/z = 912, required for [M+1]⁺: 912

### Step 5: S³⁴-(1-(2-(2-(2-(2-(2-(2-(2-(2-(2-(2-(2-(2-(2-(2-(3-((S)-2-amino-3-hydroxypropanoylamino)propylcarbamoyl)ethoxy)ethoxy)ethoxy)ethoxy)ethoxy)ethoxy)ethoxy)ethoxy)et hoxy)ethoxy)ethoxy)ethoxy)ethylcarbamoyl)ethyl)2,5-dioxo-2,5-dihydropyrrol-3-yl)albumin

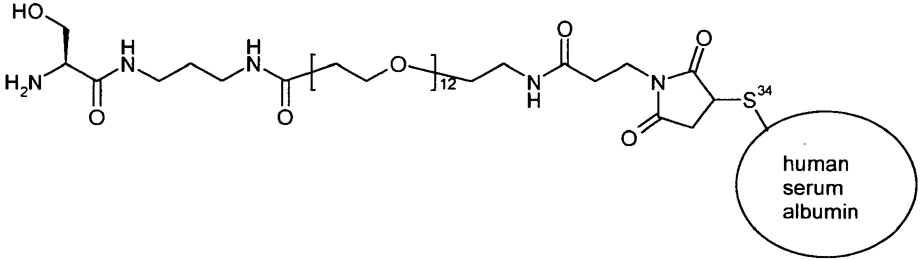

Recombinant albumin having a free cysteine group (2.2 mg, 33 nmol, commercially available at e.g. New Century Pharmaceuticals Inc.) was dissolved in water (0.200 ml). A solution of triethanolamine (0.0077 mg) in water (0.100 ml) and a solution of 3-methylthiopropan-1-ol (0.153 mg, 1025 nmol) in water (0.1 mmol) were added. A solution of sodium periodate (0.019 mg, 88 nmol) in water (0.100 ml) was added. The reaction mixture was kept in the dark at room temperature for 40 min. The buffer in of the reaction mixture was changed to a solution of triethanolamine (0.154 ml) in water (0.200 ml) by repeated ultracentrifugation in an Amicon Ultra centrifugation device with a molecular cut off of 10 kDa at a speed of 4000 rpm for 6 min.

### Step 6: Step 5: S³⁴-(1-(2-(2-(2-(2-(2-(2-(2-(2-(2-(2-(2-(2-(2-(2-(3-(2-oxoacetylamino)propylcarbamoyl)ethoxy)ethoxy)ethoxy)ethoxy)ethoxy)ethoxy)ethoxy)ethoxy)ethoxy )ethoxy)ethoxy)ethoxy)ethylcarbamoyl)ethyl)2,5-dioxo-2,5-dihydropyrrol-3-yl)albumin (Inter mediate 7)

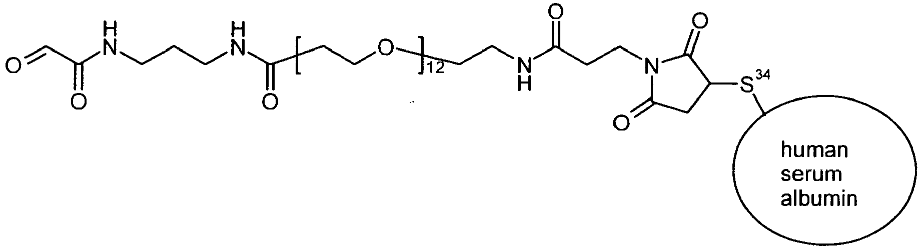

*S*³⁴-(1-(2-(2-(2-(2-(2-(2-(2-(2-(2-(2-(2-(2-(2-(2-(3-((*S*)-2-amino-3-hydroxypropanoylamino)propylcarbamoyl)ethoxy)ethoxy)ethoxy)ethoxy)ethoxy)ethoxy)ethoxy)ethoxy)ethoxy)-ethoxy)ethoxy)ethoxy)ethylcarbamoyl)ethyl)2,5-dioxo-2,5-dihydropyrrol-3-yl)albumin (21 mg, 312 nmol) was dissolved in water (0.800 ml). A solution of triethanolamine (0.077 mg) in water (1.00 ml) and a solution of 3-methylthiopropan-1-ol (1.53 mg, 10250 nmol) in water (1.0 ml) were added. A solution of sodium periodate (0.19 mg, 880 nmol) in water (1.00 ml) was added. The reaction mixture was kept in the dark at room temperature for 40 min. The buffer in of the reaction mixture was changed to a solution of triethanolamine (0.77 ml) in water (1.00 ml) by repeated ultracentrifugation in an Amicon Ultra centrifugation device with a molecular cut off of 10 kDa at a speed of 4000 rpm for 6 min._The material was used directly in an oxime formation reaction.

### Intermediate 8: periodate-oxidized colominic acid

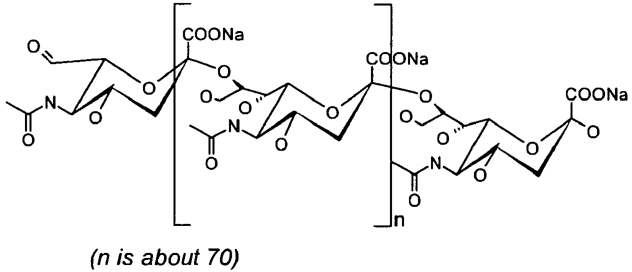

### Step 1: Fractionation of colominic acid

The colominic acid used was the commercial compound from Sigma-Aldrich (sodium salt). In order to get a more homogenous material (regarding its molecular weight), it was fractionated on an ion exchange column according to WO 2008/074032. The fraction corresponding to a molecular weight of about 20kD was used in the subsequent experiments.

### Step 2: Sodium periodate oxidation of 20kD colominic acid:

To a solution of 20kD colominic acid (as obtained in step 1 (40mg in 2.24ml H₂O), was added a sodium periodate solution (0.96mg in 2.244ml H2O).

The reaction was incubated for 15min at 23°C in the dark.

The excess of periodate was quenched by 3-methylhio-1-propanol (4.7µl). The reaction was further incubated for 2h at 23°C.

The reagents were eliminated by ultra filtration on Millipore Ultra, 5kD cut-off. Several rounds of dilution in water were done.

The resulting material was lyophilized.

### Intermediate 9:

*C*³⁴-(1-(5-(2-(ω-(3-(4-(formyl)benzoylamino)propylcarbamoylmethyl)3 kDa PEGyl)-ethylcarbamoyl)pentyl)2,5-dioxopyrrolidin-3-yl)albumin

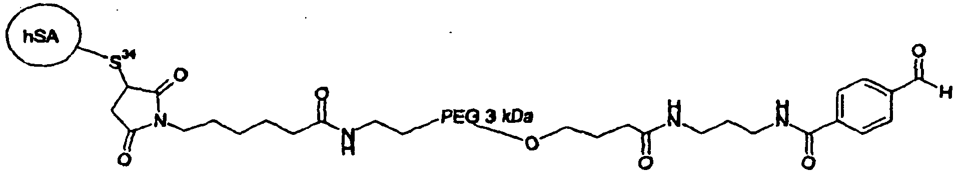

### Step 1:

### N-[3-(tert-Butoxycarbonyalamino)propyl]-4-formylbenzamide

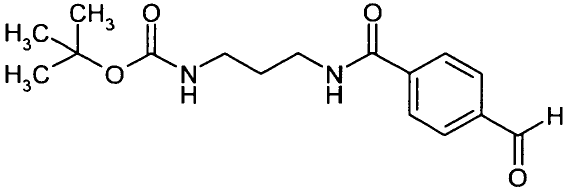

4-Formylbenzoic acid 2,5-dioxopyrrolidin-1-yl ester (1.56 g, 6.31 mmol) was added to a solution of commercially available 3-aminopropylcarbamoic acid tert-butyl ester (1.10 ml, 6.31 mmol) and ethyldiisopropylamine (2.16 ml, 12.62 mmol) in dichloromethane (25 ml). The reaction mixture was stirred at room temperature for 24 h. Dichloromethane (100 ml) was added. The mixture was washed with a 10% aqueous solution of sodium hydrogen sulphate (70 ml). The aqueous phase was extracted with dichloromethane (50 ml). The combined organic layers were washed with brine (100 ml) and were dried over magnesium sulphate. The solvent was removed in vacuo. The material was purified by flash chromatography on silica (90 g), using a mixture of ethyl acetate/heptane (3:1) as eluent to give 1.05 g of N-[3-(tert-butoxycarbonyalmaino)propyl]-4-formylbenzamide.

MS: m/z = 329, required for [M+Na]⁺: 329.

¹H-NMR (CDCl₃): δ 1.46 (s, 9 H); 1.75 (quintet, 2 H); 3.28 (t, 2 H); 3.54 (t, 2 H); 4.84 (br, 1 H); 7.60 (br, 1 H); 7.96 (d, 2 H); 8.03 (d, 2 H); 10.09 (s, 1 H).

### Step 2:

### N-[3-Aminopropyl]-4-formylbenzamide

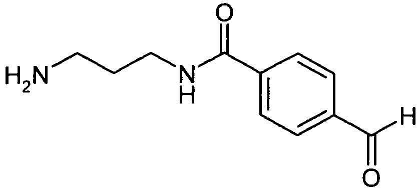

Trifluoroacetic acid (10 ml) was added to a solution of *N*-[3-(*tert-*butoxycarbonyalmaino)propyl]-4-formylbenzamide (1.1 g, 3.43 mmol) in dichloromethane (10 ml). The reaction mixture was stirred at room temperature for 1.5 h. The solvent was removed in vacuo. The residue was redissolved in dichloromethane (50 ml). The solvent was removed in vacuo. The residue was redissolved in dichloromethane (50 ml). The solvent was removed in vacuo. The residue was redissolved in dichloromethane (50 ml). The solvent was removed in vacuo to give 1.76 g of the trifluoroacetate salt of *N*-[3-aminopropyl]-4-formylbenzamide.

¹H-NMR (CDCl₃): δ 1.46 (s, 9 H); 2.07 (br, 2 H); 3.71 (q, 2 H), 4.20 (br, 2 H); 7.95 (d, 2 H); 8.01 (d, 2 H); 10.11 (s, 1 H).

### Step 3:

### N-(3-(ω-(2-(5-(2,5-Dioxo-2,5-dihydropyrrol-1-yl)hexanoylamino)ethyl)3 kDa PEGylacetylamino)propyl)-4-formylbenzamide

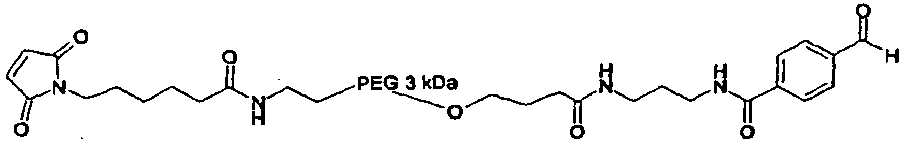

Trifluoroacetate salt of *N*-[3-aminopropyl]-4-formylbenzamide (57 mg, 0.178 mmol) was added to a solution of commercially available (e.g. Rapp Polymere GmbH, Germany) (ω-(2-(6-(2,5-dioxo-2,5-dihydropyrrol-1-yl)hexanoylamino)ethyl)3 kDa PEGyl)acetic acid 2,5-dioxopyrrolidin-1-yl ester (500 mg, 0.149 mmol) in dichloromethane (4 ml). Ethyldiisopropylamine (0.893 ml, 5.25 mmol) was added. The pH was checked to be approximately pH 10-11 by use of a pH-strip. The reaction mixture was stirred for 1 h. Ether (70 ml) was added. The mixture was left at room temperature for 1 h in order to let the formed precipitate to grow old. The precipitate was isolated by filtration. It was suspended in ether (50 ml). The precipitate was isolated by filtration to give 475 mg of N-(3-(ω-(2-(5-(2,5-dioxo-2,5-dihydropyrrol-1-yl)hexanoylamino)ethyl)3 kDa PEGylacetylamino)propyl)-4-formylbenzamide. In accordance with the expectation for the desired product, the ¹H-NMR spectrum in CDCl₃ showed the presence of a maleimide group as well as a para-substituted aromatic ring and an aldehyde group.

### Step 4:

A solution of *N*-(3-(ω-(2-(5-(2,5-dioxo-2,5-dihydropyrrol-1-yl)hexanoylamino)ethyl)3 kDa PEGylacetylamino)propyl)-4-formylbenzamide (1.6 mg, 452 nmol) in a buffer (1.6 ml) consisting of 25 mM HEPES which had been adjusted to pH 7.00 by addition of 1 N sodium hydroxide was added to a solution of recombinant human serum albumin (hSA, 15 mg, 226 nmol) with free cysteine in a buffer (13, 4 ml) consisting of 25 mM HEPES which had been adjusted to pH 7.00 by addition of 1 N sodium hydroxide. The reaction mixture was gently shaken at 300 rpm at 20-22 °C for 16 h. The material was put into an Amicon ultracentrifugation device with a cut off of 10 kDa. A buffer (15 ml) consisting of 25 mM TRIS which had been adjusted to pH 8.00 by addition of 1 N hydrochloric acid was added. This solution was subjected to a centrifugation at 4000 rpm for 10 min. A buffer (15 ml) consisting of 25 mM TRIS which had been adjusted to pH 8.00 by addition of 1 N hydrochloric acid was added. This solution was subjected to a centrifugation at 4000 rpm for 10 min. The material was subjected to an anion exchange chromatography on a MonoQ column with a bed size of approx. 8 ml using a gradient of 0-75% of a buffer consisting of 25 mM TRIS and 2 M NaCl which had been adjusted to pH 8.00 in a buffer consisting of 25 mM TRIS which had been adjusted to pH 8.00 over 30 CV at a flow of 4 ml/min. The application of the sample to the column was done with a flow of 0.5 ml/min. The fractions containing material which SDS-PAGE analysis was in accordance with C³⁴-(1-(5-(2-(ω-(3-(4-(formyl)benzoylamino)propylcarbamoylmethyl)3 kDa PEGyl)ethylcarbamoyl)pentyl)2,5-dioxopyrrolidin-3-yl)albumin were combined. The combined fractions were subjected to a size exclusion chromatography using 53 ml of a Superdex G25 material and a buffer consisting of 25 mM ammonium hydrogencarbonate at a flow of 7 ml/min. The fractions containing material which SDS-PAGE analysis was in accordance with C³⁴-(1-(5-(2-(ω-(3-(4-(formyl)benzoylamino)propylcarbamoylmethyl)3 kDa PEGyl)ethylcarbamoyl)pentyl)2,5-dioxopyrrolidin-3-yl)albumin were collected, combined and subjected to lyophilization to give 0.803 mg of the title compound. The yield was determined on a Nanodrop photometry apparatus at 280 nm using a molar absorbance of 4.11.

### Reaction of N^{Gln}-(3-aminoxy propyloxy) Factor VIII (Intermediate 2) with aldehydes (Intermediates 3, 4, 5, 6, 7, 8 and 9)

Intermediate 2 as prepared above was reacted with a number of aldehydes in oximation reactions, to form Factor VIII derivatives.

### Example 1 - oximation of Intermediate 2 with 3-(mPEGyl)propanal to give N^{Gln}-(3-(3-(mPEGyl)-propylideneaminoxy)propyloxy)FVIII

The mPEGyl is polydisperse and has a molecular weight of approximately 20kDa. The following solutions were prepared:
- Intermediate 2 solution: 1.74mg/ml in buffer D;
- 3-(mPEGyl)propanal (20kDa)(CH₃O(CH₂CH₂O)ₙ-CH₂CH₂CHO ME-200AL from NOF): 12.9mg/ml in buffer E; and
- 4-hydroxybenzaldehyde (MW=122.13). Solution 13.8mg/ml in buffer E.

To the solution of Intermediate 2 (77.6µl, 135µg) was added buffer E (2.4µl) and the 3-(mPEGyl)propanal solution in buffer E (595µl, 7.7mg). The reaction mixture was incubated for 3h at 25°C. 4-hydroxybenzaldehyde solution (32.8µl, 453µg) was then added (capping of unreacted hydroxylamine moieties). The reaction mixture was further incubated for 3h at 25°C.

The product was purified by ion exchange as follows. The reaction mixture was diluted eight times in buffer C, and applied on two ion exchange Vivapure Q Mini M devices (VivaScience product number VS-IX01QM24, Vivascience AG, Germany) which had been previously equilibrated with buffer C. After two washing steps with the same buffer, the reaction product was eluted with the elution buffer D.

The protein concentration was estimated by measurement of absorption at 280nm (E_{1%}=14.6 Lg⁻¹cm⁻¹)(Nanodrop ND-1000, Nanodrop Technologies, Inc, USA) giving an estimated protein recovery of 83%.

The product was run on SDS polyacrylamide gel electrophoresis using NuPage 7% Tris- acetate gel (Invitrogen EA03555BOX) according to manufacturer instructions (70min at 150V). The gels were silver stained (Invitrogen LC6070). Standard proteins were from Invitrogen (HiMark HMW Standard LC5688).

The SDS gel shows that the heavy chain is the most heavily modified. Several bands of higher MW appear, as expected. The band of highest MW appears at around 400kDa.

### The product was subjected to thrombin digestion:

Thrombin (Human thrombin, Roche diagnostica) was solubilised at 20U/ml in H₂O, and further diluted to 2U/ml in buffer A. To a solution of the product of example 1 (2.9µl, 1.2µg) was added buffer A (8.9µl) and the thrombin solution (0.24µl). The reaction mixture was incubated at 37°C for 15min. A thrombin digestion of FVIII was run in parallel. The reaction mixtures were analyzed by HPLC on a Zorbax 300SB-C18, 0.21x15cm, 5µ. The eluents were A: 0.1% TFA in water, and B: 0.07% TFA in acetonitrile. The flow was 0.2ml/min, the temperature 40°C. The gradient was as follows: from 0 to 15% over 2min, from 15 to 80% over 21 min, from 80 to 100%B over 10min. The detection was done by UV (λ=280nm).

The chromatograms obtained showed that the peak corresponding to the A1 domain in thrombin digested FVIII (blue trace) almost disappeared in the thrombin digest of the pegylated FVIII of example 1 (red trace). Thus, the A1 domain was indeed the most heavily modified.

### Example 2 - Oximation of Intermediate 2 with Intermediate 3 to give NG^{Gln}-(3-(4-(1-(3-((omega-(17-(carboxy)heptadecanoylamino)5 kDa PEGyl)carbamoyl)benzyl)1,2,3-triazol-4-yl)butylideneaminoxy)propyloxy)FVIII

The following solutions were prepared:
- Intermediate 2: 2.36mg/ml in buffer D;
- Intermediate 3 (MW: 5600): 1.79mM in buffer E;
- 4-hydroxybenzaldehyde solution: 17.3mg/ml in buffer E; and
- methoxylamine hydrochloride: 11mg/ml in buffer E.

To the solution of Intermediate 3 (500µl, 848nmoles) in buffer E was added buffer E (644.3µl) and the Intermediate 2 solution in buffer E (105.9µl, 250µg). The reaction mixture was incubated for 3h at 25°C. 4-hydroxybenzaldehyde solution (50pl, 865µg) was then added (capping of unreacted hydroxylamine moieties). The reaction mixture was further incubated for 1 h at 25°C. The aldehyde in excess was quenched by addition of methoxylamine hydrochloride (62.5µl, 687µg). The reaction mixture was further incubated for 30min at 25°C.

The product was purified by ion exchange as follows. The salt concentration was lowered to below 25mM salt concentration by successive dilution (with buffer buffer C) and upconcentration steps on Amicon Ultra devices (50kDa cut-off) (Millipore Corp., USA). The solution obtained was applied on two ion exchange Vivapure Q Mini M devices (VivaScience product number VS-IX01QM24, Vivascience AG, Germany) which had been previously equilibrated with buffer C. After two washing steps with the same buffer, the reaction product was eluted with the elution buffer D.

The protein concentration was estimated by measurement of absorption at 280nm (E_{1%}=14.6 Lg⁻¹cm⁻¹)(Nanodrop ND-1 000, Nanodrop Technologies, Inc, USA) giving an estimated protein recovery of 73%.

The product was run on SDS polyacrylamide gel electrophoresis using NuPage 7% Tris- acetate gel (Invitrogen EA03555BOX) according to manufacturer instructions (70min at 150V). The gels were silver stained (Invitrogen LC6070). Standard proteins were from Invitrogen (HiMark HMW Standard LC5688).

The SDS gel shows that the heavy chain is again the most heavily modified. Several bands of higher MW appear. The band of highest MW appears at around 120kDa.

The product was also run on HPLC, on a Vydac C4, 0.21x5cm, 5µ (Vydac n°: 214TP5205).

The eluents were A: 0.1 % TFA in water, and B: 0.07% TFA in acetonitrile. The flow was 0.2ml/min, the temperature 40°C. The gradient was as follows: from 30 to 40% over 3min, from 40 to 50% over 60min, from 50 to 100%B over 12.5min. The detection was done by UV (λ=280nm) and by fluorescence (λ Exc=280nm, Em=348nm).

The chromatograms obtained confirmed that the heavy chain was indeed the most heavily modified.

### Example 3 - oximation of Intermediate 2 with Intermediate 4

The following solutions were prepared:
- Intermediate 2: 2.36mg/ml in buffer D
- Intermediate 4 (MW= 122.1): 5.55mg/ml
- Methylhydroxylamine hydrochloride (MW=83.5). 10.2mg/ml in buffer E

The Intermediate 2 solution (106.7µl, 250µg) and the solution of the aldehyde reagent Intermediate 4 obtained above(1.144ml, 833.8µg) were mixed and incubated for 3h at 25°C. Capping of possibly remaining free aminoxy groups was done by addition of 4-hydroxy benzaldehyde solution (63µl, 350µg), and the resulting mixture was incubated for 1 h at 25°C. The aldehyde in excess was quenched by addition of methyl hydroxylamine hydrochloride (62.5µl, 638µg). The reaction mixture was further incubated for 30min at 25°C.

The product was purified by ion exchange as follows. The reaction mixture was diluted by addition of buffer C (9.625ml). The solution obtained was applied on two ion exchange Vivapure Q Maxi M devices (VivaScience product number VS-IX20QM08, Vivascience AG, Germany) which had been previously equilibrated with buffer C. After two washing steps with the same buffer, the reaction product was eluted with the elution buffer D.

The protein concentration was estimated by measurement of absorption at 280nm (E_{1%}=14.6 Lg⁻¹cm⁻¹)(Nanodrop ND-1 000, Nanodrop Technologies, Inc, USA) giving an estimated protein recovery of 96%.

The product was run on SDS polyacrylamide gel electrophoresis using NuPage 7% Tris- acetate gel (Invitrogen EA03555BOX) according to manufacturer instructions (70min at 150V). The gels were silver stained (Invitrogen LC6070). Standard proteins were from Invitrogen (HiMark HMW Standard LC5688):

The product was run on HPLC, on a Vydac C4, 0.21x5cm, 5µ (Vydac n°: 214TP5205). The eluents were A: 0.1% TFA in water, and B: 0.07% TFA in acetonitrile. The flow was 0.2ml/min, the temperature 40°C. The gradient was as follows: from 30 to 40%B over 3min, from 40 to 50%B over 60min, from 50 to 100%B over 12.5min. The detection was done by UV (λ=280nm) and by fluorescence (λ Exc=280nm, Em=348nm).

The chromatograms obtained showed that the heavy chain was the most heavily modified, as expected.

### Example 4 - oximation of Intermediate 2 with Intermediate 5 to give

### N^{Gln}-(3-(4-(2-(2-(2-(2-(2-(2-(2-(2-(2-(2-(2-(2-(biotinylamino)ethoxy)ethoxy)ethoxy)-ethoxy)ethoxy)ethoxy)ethoxy)ethoxy)ethoxy)ethoxy)ethoxy)ethylcarbamoyl)-benzylideneiminoxy)propyloxy)FVIII

The following solutions were prepared.
- Intermediate 2: 1.74 mg/ml in 20mM imidazol buffer containing 0.02% Tween 80, 10% v/v glycerol, 1 M NaCl, 10mM CaCl2, pH 7.4;
- Intermediate 5: 1.28mg/ml in buffer G; and
- Methylhydroxylamine hydrochloride: 2.84mg/ml in buffer G

To the Intermediate 2 solution (115µl, 200µg) was added buffer G (485µl), and the solution of Intermediate 5 (400µl, 513µg). The reaction mixture was incubated for 3h at 25°C. The aldehyde in excess was quenched by addition of methyl hydroxylamine hydrochloride (25µl, 71 µg). The reaction mixture was further incubated for 30min at 25°C.

The salt concentration was lowered to below 25mM salt concentration by successive dilution (with buffer C) and upconcentration steps on Amicon Ultra devices (50kDa cut-off) (Millipore Corp., USA). The solution obtained was applied on two ion exchange Vivapure Q Mini M devices (VivaScience product number VS-IX01QM24, Vivascience AG, Germany) which had been previously equilibrated with buffer C. After two washing steps with the same buffer, the reaction product was eluted with the elution buffer D.

The protein concentration was estimated by measurement of absorption at 280nm
(Nanodrop ND-1000, Nanodrop Technologies, Inc, USA) giving an estimated protein recovery of 50%.

The product was run on SDS polyacrylamide gel electrophoresis using NuPage 7% Tris-acetate gel (two identical gels run)(Invitrogen EA03555BOX) according to manufacturer instructions (70min at 150V). Standard proteins were from Invitrogen (HiMark HMW Standard LC5688).

One gel was silver stained (Invitrogen LC6070), the other gel was blotted with streptavidin-HRP (Invitrogen 43-4323).

The most strongly stained band on the blot corresponds to the heavy chain as expected.

### Example 5 - oximation of Intermediate 2 with Intermediate 7

A solution of Intermediate 2 ( 1.00 mg, 5.60 nmol) in a buffer consisting of 20 mM imidazole, 10 mM CaCl₂, 10% glycerol, and 0.02% Tween 80 was added to Intermediate 7 The reaction mixture was left at room temperature for 16 h. A 5 M aqueous solution of sodium chloride (0.046 ml). The reaction mixture was applied to a column, prepared from F25 antibody (as described in e.g. WO95/013301), which had been activated by CNBr. Unbound material was washed out. The column was washed with a buffer (5 ml) consisting of 20 mM imidazole, 10 mM CaCl₂, 0.02% Tween 80 and 650 mM NaCl, pH 7.35. Another fraction was washed out with a buffer (5 ml) consisting of 20 mM imidazole, 10 mM CaCl₂, 0.02% Tween 80 and 2.5 M NaCl, 50 %v/v ehtyleneglycol, pH 7.35. SDS-analysis indicated that an albumin-FVIII conjugate may have been washed from the column using the last buffer (20 mM imidazole, 10 mM CaCl₂, 0.02% Tween 80 and 2.5 M NaCl, 50 %v/v ehtyleneglycol, pH 7.35). The albumin-FVIII conjugate was identified on SDS-gel by bands at approx. 70 kDa and 90 kDa, corresponding to the molecular weights of the heavy and the light chain as well as bands at approximately 140 kDa, and 160 kDa, corresponding to the expected mass of albumin conjugates of the light chain and the heavy chain respectively.

### Example 6 - oximation of Intermediate 2 with Intermediate 6

### to give N^{Gln}-(3-(4-(2-(2-(2-(2-(2-(2-(2-(2-(2-(2-(2-(2-(Alexa 488-ylamino)ethoxy)-ethoxy)ethoxy)ethoxy)ethoxy)ethoxy)ethoxy)ethoxy)ethoxy)ethoxy)ethoxy)-ethylcarbamoyl)benzylideneiminoxy)propyloxy)FVIII

### Solutions:

- *N*^{Gln}-(3-aminoxy propyloxy)FVIII: 1.74 mg/ml in 20mM imidazol buffer containing 0.02% Tween 80, 10% v/v glycerol, 1M NaCl, 10mM CaCl2, pH 7.4
- Intermediate 6: 1.70mg/ml in buffer G

### Procedure:

To the N-(3-(aminoxy)propyloxy)FVIII solution (115µl, 200µg) was added buffer G (485µl), and the solution of intermediate 6 (400µl, 678µg). The reaction mixture was incubated for 3h30 at 25°C.

The aldehyde in excess was quenched by addition of methyl hydroxylamine hydrochloride (25µl, 71µg). The reaction mixture was further incubated for 45min at ambiant temperature.

### Purification:

The purification was done as in example 4.

The protein concentration was estimated by measurement of absorption at 280nm

(Nanodrop ND-1000, Nanodrop Technologies, Inc, USA) giving an estimated protein recovery of 40%.

### Example 7 - oximation of Intermediate 2 with Intermediate 8

### Solutions:

- Intermediate 2: 2.74 mg/ml in 20mM imidazol buffer containing 0.02% Tween 80, 10% v/v glycerol, 1 M NaCl, 10mM CaCl2, pH 7.4
- Methylhydroxylamine hydrochloride in solution in 100mM imidazole buffer pH6.5 containing 0.02% Tween 80, 10% v/v glycerol, 10mM CaCl2

### Procedure:

To the intermediate 2 solution (91 µl, 250µg) was added the intermediate 7 solution in buffer G (175µl, 15.7mg). The reaction mixture was incubated for 2h at 27°C.

The aldehyde in excess was quenched by addition of methyl hydroxylamine hydrochloride (12.5µl, 296µg). The reaction mixture was further incubated for 30min at 27°C

### Purification:

The purification was done as in example 4. The protein concentration was estimated by measurement of absorption at 280nm (Nanodrop ND-1000, Nanodrop Technologies, Inc, USA) giving an estimated protein recovery of 45%.

The product was run on SDS polyacrylamide gel electrophoresis using NuPage 7% Tris- acetate gel (Invitrogen EA03555BOX) according to manufacturer instructions (70min at 150V). The gels were silver stained (Invitrogen LC6070). Standard proteins were from Invitrogen (HiMark HMW Standard LC5688).

Again, the heavy chain of FVIII was the most heavily modified, the colominic acid-FVIII conjugate was identified on SDS gel by a wide and diffuse band present between 93 and about 140kD.

### Example 8: Oximation of intermediate 2 with intermediate 9

To the intermediate 2 (210µg, 1.42nmole) in solution in buffer E (44µl) was added a solution of C³⁴-(1-(5-(2-(ω-(3-(4-(formyl)benzoylamino)propylcarbamoylmethyl)3 kDa PEGyl)-ethylcarbamoyl)pentyl)2,5-dioxopyrrolidin-3-yl)albumin (100µg, 1.41nmole) in solution in buffer E (10µl). A 0.3M aniline solution in buffer E was added (2µl). The reaction was incubated overnight at 30°C.

The excess of intermediate 2 was quenched by addition of 4-hydroxybenzaldehyde (1.46µg) in water (2µl). The mixture was left for 1 h at 30°C.

The mixture was diluted 1:11 (v/v) with buffer C before purification by anion exchange on VivaPure Q mini M (Vivascience): following loading, the material was washed with buffer C, and eluted with buffer D. The eluate was then subjected to size exclusion chromatography on a Superdex 200 10/300 GL column (GE Healthcare). The flow was 0.5ml/min, and the eluent was a buffer consisting of sucrose (3g/l), histidine (1.5g/l), sodium chloride (18g/l), Tween 80 (0.1g/l), calcium chloride (0.25g/l), pH7.3.

An SDS PAGE analysis showed the presence of bands at about 166kD, 228kD and 332kD, which were tentatively assigned to coupling of respectively one, two or three C³⁴-(1-(5-(2-(ω-(3-(4-(formyl)benzoylamino)propylcarbamoylmethyl)3 kDa PEGyl)ethylcarbamoyl)-pentyl)2,5-dioxopyrrolidin-3-yl)albumin moieties on the heavy chain of FVIII.

### FVIII Chromogenic Activity Analysis - COA Test

The activity of a FVIII containing sample can be determined with a commercially available COA test (COATEST® SP FVIII, Chromogenix Art. No.: 82 4086 63).

### Determination of Pharmacokinetic parameters

15 FVIII KO mice bred at Taconic M&B weighing approximately 21.8 g were used for the study. The mice were dosed the compounds as a single injection in the tail vein and were anaesthetized by Isofluran/O₂/N₂O for blood sampling. Blood from two or three mice pr time-point was sampled at 0.08, 0.33, 1, 3, 7, 16, 24, 48, 64 h post administration from the orbital plexus. 4 droplets of blood were sampled from the eye by use of a 10 µl capillary glass tube. After the third blood sample, the mice were killed by cervical dislocation. 45 µl of blood was transferred to Eppendorf tubes containing 5 µl of sodium-citrate (0.13 M). 200 µl FVIII coatest SP buffer was added and diluted blood was centrifuged at 4000 g for 5 minutes at room temperature. The supernatant was analysed by means of ELISA antigen and chromogenic activity analysis.

**Table 1 compounds and doses**

| Compound Description | Example no | Dose | Dose volume | Conc.* |
|---|---|---|---|---|
| | | IU/kg | (ml/kg) | (IU/ml) |
| Product of reaction of interme-diate 2 and intermediate 3 | 2 | 280 | 5 | 56 |
| Product of reaction of interme-diate 2 and intermediate 4 | 3 | 280 | 5 | 56 |
| Product of reaction of interme-diate 2 with 3-(mPEGyl)propanal | 1 | 280 | 4.30 | 65.1 |

**Table 2 PK parameters as obtained by NCA**

| | | Cmax | AUC | AUC extrap | T½ | Cl | Vss | MRT |
|---|---|---|---|---|---|---|---|---|
| Assay | Example | (IU/I) | (h*IU/I) | (%) | (h) | (ml/h/kg) | (ml/kg) | (h) |
| FVIII COA | 1 | 2945 | 36751 | 1 | 11 | 7,6 | 116 | 15 |
| FVIII COA | 2 | 2504 | 37258 | 5 | 13 | 6,9 | 137 | 20 |
| FVIII COA | 3 | 4534 | 46025 | 2 | 9,3 | 10 | 121 | 12 |
| FVIII COA | BDD-FVIII | 2740 | 26000 | 3 | 7,8 | 11 | 117 | 11 |
| FVIII ELISA | 1 | 2557 | 30635 | 8 | 13 | 9,1 | 160 | 17 |
| FVIII ELISA | 2 | 2750 | 39054 | 4 | 13 | 7,2 | 128 | 18 |
| FVIII ELISA | 3 | 3020 | 27927 | 3 | 9,6 | 10 | 126 | 13 |
| FVIII ELISA | BDD-FVIII | 1990 | 20000 | 10 | 8,2 | 14 | 159 | 12 |

A prolongation or slight prolongation of the terminal half-life (9.3-13 h) is observed after i.v. administration of all three compounds as compared to B-Domain Deleted FVIII all (BDD-FVIII) (7.8-8.2 h) when measured by both FVIII chromogenic activity and FVIII ELISA. The mean residence time (MRT) was similarly increased of the three compounds (12-20 h) as compared to BDD-FVIII (11-12 h). Furthermore, the clearance of the compounds was reduced (6.9-10 ml/h/kg) as compared to BDD-FVIII (11-14 ml/h/kg).

## Claims

1. A Factor VIII derivative of formula (I): 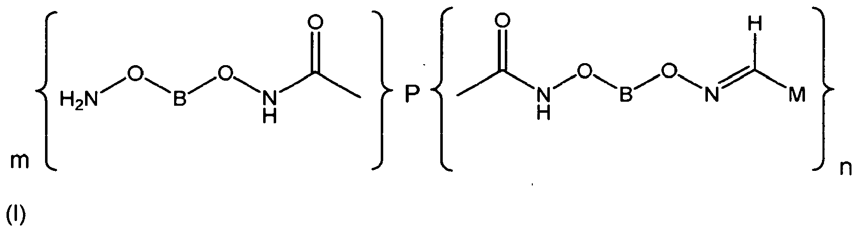 wherein:
B represents C₂ to C₁₀ alkylene;
m represents 0 or an integer from 1 to 19, n represents an integer from 1 to 20, and the sum of m and n is from 1 to 20;
P represents a mono or polyradical of Factor VIII obtained by removing m + n carbamoyl groups from the side chains of glutamine residues in Factor VIII; and
M represents a moiety (M¹) that increases the plasma half-life of the Factor VIII derivative or a reporter moiety (M²);
or a pharmaceutically acceptable salt thereof.

2. A Factor VII derivative according to claim 1, wherein said moiety M¹ comprises one or more polyethyleneglycol (PEG) moieties, polypeptides or plasma protein binders.

3. A Factor VIII derivative according to claim 2, wherein said peptide is albumin, or an antibody or fragment thereof.

4. A Factor VIII derivative according to claim 2, wherein said plasma protein binder is an albumin binder.

5. A Factor VIII derivative according to claim 2, wherein said moiety M¹ comprises a polyethyleneglycol (PEG) moiety.

6. A Factor VIII derivative according to claim 1, wherein:
B represents C₂ to C₄ alkylene;
m represents 0 or an integer from 1 to 5, n represents an integer from 1 to 6, and the sum of m and n is from 1 to 6; and
M represents a moiety (M¹) that comprises a polyethyleneglycol (PEG) moiety and/or an albumin binder.

7. A Factor VIII derivative according to claim 1, wherein:
B represents C₂ to C₄ alkylene;
m represents 0 or an integer from 1 to 5, n represents an integer from 1 to 6, and the sum of m and n is from 1 to 6; and
M represents a moiety (M²) that comprises biotin or a fluorescent marker.

8. A Factor VIII derivative according to claim 6 or 7 wherein B represent C₃ alkylene.

9. A pharmaceutical composition comprising a Factor VIII derivative as defined in any one of the preceding claims and a pharmaceutically acceptable carrier or diluent.

10. A Factor VIII derivative as defined in any one of claims 1 to 8 for use in the treatment of the human or animal body by therapy.

11. A Factor VIII derivative according to claim 10 for use in the treatment of Haemophilia A.

12. A Factor VIII derivative of formula (II) 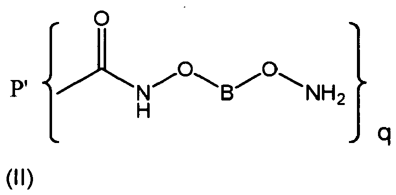 wherein:
B represents a C₂ to C₁₀ alkylene;
q represents an integer from 1 to 20; and
P' represents a mono or polyradical of Factor VIII obtained by removing q carbamoyl groups from the side chains of glutamine residues in Factor VIII;
or a pharmaceutically acceptable salt thereof.

13. A method for preparing a Factor VIII derivative of formula (II) as defined in claim 12, which method comprises reacting Factor VIII with a compound of formula (III):
H₂N-O-B-O-NH₂ (III)
in the presence of a transglutaminase, wherein B is as defined in any one of claims 1 to 8.

14. A method according to claim 13, wherein the transglutaminase is *Streptomyces mobaraense* transglutaminase.

15. A method for preparing a Factor VIII derivative of formula (I) as defined in any one of claims 1 to 8 comprising reacting a Factor VIII derivative of formula (II) as defined in claim 12 with an aldehyde of formula (IV): 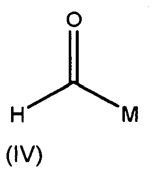 wherein M is as defined in any one of claims 1 to 6, or 7.

## Patentansprüche

1. Faktor-VIII-Derivat der Formel (I): 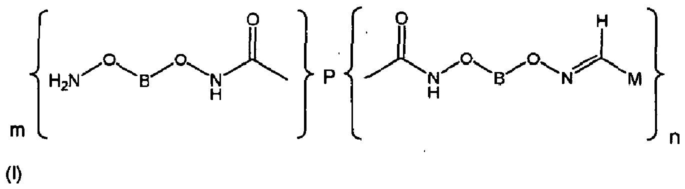 wobei:
B C₂- bis C₁₀-Alkylen darstellt;
m 0 oder eine ganze Zahl von 1 bis 19 darstellt, n eine ganze Zahl von 1 bis 20 darstellt und die Summe von m und n 1 bis 20 beträgt;
P einen Mono- oder Polyrest von Faktor VIII darstellt, der durch Entfernen von m+n Carbamoylgruppen von den Seitenketten von Glutaminresten in Faktor VIII erhalten wird; und
M eine Einheit (M¹), die die Plasmahalbwertszeit des Faktor-VIII-Derivats erhöht, oder eine Reportereinheit (M²) darstellt;
oder ein pharmazeutisch verträgliches Salz davon.

2. Faktor-VIII-Derivat nach Anspruch 1, wobei die Einheit M¹ eine oder mehrere Polyethylenglycol(PEG)-Einheiten, Polypeptide oder Plasmaproteinbindemittel umfasst.

3. Faktor-VIII-Derivat nach Anspruch 2, wobei das Peptid Albumin oder ein Antikörper oder Fragment davon ist.

4. Faktor-VIII-Derivat nach Anspruch 2, wobei das Plasmaproteinbindemittel ein Albuminbindemittel ist.

5. Faktor-VIII-Derivat nach Anspruch 2, wobei die Einheit M¹ eine Polyethylenglycol(PEG)-Einheit umfasst.

6. Faktor-VIII-Derivat nach Anspruch 1, wobei:
B C₂- bis C₄-Alkylen darstellt;
m 0 oder eine ganze Zahl von 1 bis 5 darstellt, n eine ganze Zahl von 1 bis 6 darstellt und die Summe von m und n 1 bis 6 beträgt; und
M eine Einheit (M¹) darstellt, die eine Polyethylenglycol(PEG)-Einheit und/oder ein Albuminbindemittel umfasst.

7. Faktor-VIII-Derivat nach Anspruch 1, wobei:
B ein C₂- bis C₄-Alkylen darstellt;
m 0 oder eine ganze Zahl von 1 bis 5 darstellt, n eine ganze Zahl von 1 bis 6 darstellt und die Summe von m und n 1 bis 6 beträgt; und
M eine Einheit (M²) darstellt, die Biotin oder einen Fluoreszenzmarker umfasst.

8. Faktor-VIII-Derivat nach Anspruch 6 oder 7, wobei B C₃-Alkylen darstellt.

9. Arzneimittel, umfassend ein wie in einem der vorangehenden Ansprüche definiertes Faktor-VIII-Derivat und einen pharmazeutisch verträglichen Träger oder ein ebensolches Verdünnungsmittel.

10. Faktor-VIII-Derivat, wie in einem der Ansprüche 1 bis 8 definiert, zur Verwendung bei der therapeutischen Behandlung des menschlichen oder tierischen Körpers.

11. Faktor VIII-Derivat nach Anspruch 10 zur Verwendung bei der Behandlung von Hämophilie A.

12. Faktor-VIII-Derivat der Formel (II) 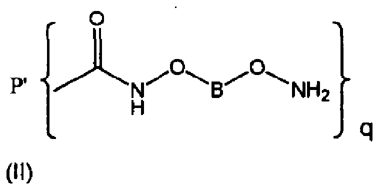 wobei:
B C₂- bis C₁₀-Alkylen darstellt;
q eine ganze Zahl von 1 bis 20 darstellt;
P' einen Mono- oder Polyrest von Faktor VIII darstellt, der durch Entfernen von q Carbamoylgruppen von den Seitenketten von Glutaminresten in Faktor VIII erhalten wird;
oder ein pharmazeutisch verträgliches Salz davon.

13. Verfahren zur Herstellung eines wie in Anspruch 12 definierten Faktor-VIII-Derivats der Formel (II), wobei das Verfahren das Umsetzen von Faktor VIII mit einer Verbindung der Formel (III):
**H₂N-O-B-O-NH₂** (III)
in der Gegenwart einer Transglutaminase umfasst, wobei B wie in einem der Ansprüche 1 bis 8 definiert ist.

14. Verfahren nach Anspruch 13, wobei die Transglutaminase Transglutaminase von *Streptomyces mobaraense* ist.

15. Verfahren zur Herstellung eines wie in einem der Ansprüche 1 bis 8 definierten Faktor-VIII-Derivats der Formel (I), umfassend das Umsetzen eines wie in Anspruch 12 definierten Faktor-VIII-Derivats der Formel (II) mit einem Aldehyd der Formel (IV): 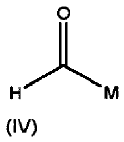 wobei M wie in einem der Ansprüche 1 bis 6 oder 7 definiert ist.

## Revendications

1. Dérivé du Facteur VIII de formule (I) 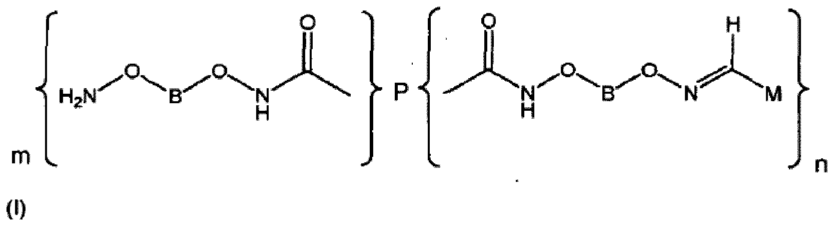 dans laquelle
B représente un radical alkylène en C₂ à C₁₀;
m représente 0 ou est un entier de 1 à 19, n représente un entier de 1 à 20, et la somme de m et de n est comprise entre 1 et 20 ;
P représente un mono- ou un polyradical du Facteur VIII, obtenu par élimination de m + n groupes carbamoyle à partir des chaînes latérales des résidus de glutamine se trouvant dans le Facteur VIII ; et
M représente un fragment (M¹) qui augmente la demi-vie plasmatique du dérivé du Facteur VIII, ou un fragment rapporteur (M²);
ou l'un de ses sels pharmaceutiquement acceptables.

2. Dérivé du Facteur VIII selon la revendication 1, dans lequel ledit fragment M¹ comprend un ou plusieurs fragments polyéthylèneglycol (PEG), polypeptides ou sites de liaison des protéines plasmatiques.

3. Dérivé du Facteur VIII selon la revendication 2, dans lequel ledit peptide est l'albumine ou un anticorps ou un fragment de cette dernière.

4. Dérivé du Facteur VIII selon la revendication 2, dans lequel ledit site de liaison des protéines plasmatiques est un site de liaison de l'albumine.

5. Dérivé du Facteur VIII selon la revendication 2, dans lequel ledit fragment M¹ comprend un fragment polyéthylèneglycol (PEG).

6. Dérivé du Facteur VIII selon la revendication 1, dans lequel :
B représente un radical alkylène en C₂ à C₄ ;
m représente 0 ou est un entier de 1 à 5, n représente un entier de 1 à 6, et la somme de m et de n vaut de 1 à 6 ; et
M représente un fragment (M¹) qui comprend un fragment polyéthylèneglycol (PEG) et/ou un site de liaison de l'albumine.

7. Dérivé du Facteur VIII selon la revendication 1, dans lequel :
B représente un radical alkylène en C₂ à C₄ ;
m représente 0 ou un entier de 1 à 5, n représente un entier de 1 à 6, et la somme de m et de n vaut de 1 à 6 ; et
M représente un fragment (M²) qui comprend de la biotine ou un marqueur fluorescent.

8. Dérivé du Facteur VIII selon la revendication 6 ou 7, dans lequel B représente un radical alkylène en C₃.

9. Composition pharmaceutique comprenant un dérivé du Facteur VIII tel que défini dans l'une quelconque des revendications précédentes et un support ou un diluant pharmaceutiquement acceptable.

10. Dérivé du Facteur VIII tel que défini dans l'une quelconque des revendications 1 à 8, pour utilisation dans le traitement, par une thérapie, du corps humain ou animal.

11. Dérivé du Facteur VIII selon la revendication 10, pour utilisation dans le traitement de l'hémophilie A.

12. Dérivé du Facteur VIII de formule (II) 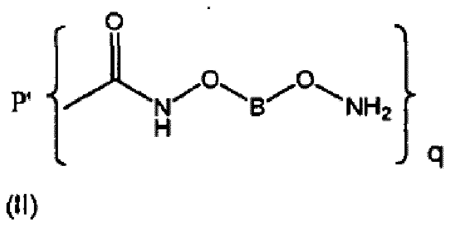 dans laquelle :
B représente un radical alkylène en C₂ à C₁₀ ;
q représente un entier de 1 à 20 ; et
P' représente un mono- ou un polyradical du Facteur VIII, obtenu par élimination de q groupes carbamoyle à partir des chaînes latérales des résidus de glutamine se trouvant dans le Facteur VIII ;
ou l'un de ses sels pharmaceutiquement acceptables.

13. Procédé de préparation d'un dérivé du Facteur VIII de formule (II) tel que défini dans la revendication 12, lequel procédé comprend la réaction du Facteur VIII avec un composé de formule (III) :
H₂N-O-B-O-NH₂ (III)
en présence d'une transglutaminase, B étant tel que défini dans l'une quelconque des revendications 1 à 8.

14. Procédé selon la revendication 13, dans lequel la transglutaminase est une transglutaminase de *Streptomyces mobaraense.*

15. Procédé de préparation d'un dérivé du Facteur VIII de formule (I) tel que défini dans l'une quelconque des revendications 1 à 8, comprenant la réaction d'un dérivé du Facteur VIII de formule (II) tel que défini dans la revendication 12 avec un aldéhyde de formule (IV) : 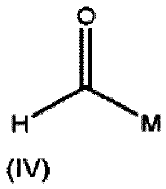 dans laquelle M est tel que défini dans l'une quelconque des revendications 1 à 6 ou 7.
